# EUROPEAN PATENT APPLICATION

(11) **EP 3 797 792 A1**
(43) Date of publication of application: **31.03.2021**
(21) Application number: 20190885.2
(22) Date of filing: 28.02.2018
(51) Int. Cl.: A61K 39/395, C07K 16/24, C07K 16/28, C07K 16/10

(54) **FORMULATIONS OF ANTI-GM-CSFRALPHA MONOCLONAL ANTIBODY**

(30) Priority: 01.03.2017 US 201762465269 P
(62) Divisional of application: 18714973.7
(71) Applicant: MedImmune Limited, Cambridge Cambridgeshire CB21 6GH (GB)
(72) Inventor: LOBO, Brian Andrew, Gaithersburg, MD 20878 (US); SHAH, Ambarish Umakant, Gaithersburg, MD 20878 (US); SHARMA, Monika Sood, Gaithersburg, MD 20878 (US); GOLDBERG, Deborah Sweet, Gaithersburg, MD 20878 (US)
(74) Representative: Grünecker Patent- und Rechtsanwälte PartG mbB

(57) **Abstract**

The present invention provides a formulation comprising: (i) a monoclonal antibody; and (ii) an ionic excipient; wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 6.5.

SEQUENCE LISTING

<110>  MedImmune Limited

<120>  FORMULATIONS OF MONOCLONAL ANTIBODIES

<130>  EP127876I_SZ382

<140>  not yet assigned
<141>  2018-02-28

<150>  EP18714973.7
<151>  2018-02-28

<150>  PCT/EP2018/054960
<151>  2018-02-28

<150>  US 62/465,269
<151>  2017-03-01

<160>  10

<170>  PatentIn version 3.5

<210>  1
<211>  1074
<212>  DNA
<213>  Artificial Sequence

<220>
<223>  Coding strand for CAT-354 light chain DNA sequence

<400>  1
tcagatcgcc tggagacgcc atccacgctg ttttgacctc catagaagac accgggaccg      60

atccagcctc cgcggccggg aacggtgcat tggaacgcgg attccccgtg ccaagagtga     120

ctcaccgtcc ttgacacgaa gctattcgaa ctacagttac tgagcacaca ggacctcacc     180

atgggatgga gctgtatcat cctcttcttg gtagcaacag ctacaggtgt ccactcctcc     240

tatgtgctga ctcagccacc ctcggtgtca gtggccccag gaaagacggc caggattacc     300

tgtgggggaa acatcattgg aagtaaactt gtacactggt accagcagaa gccaggccag     360

gcccctgtgc tggtcatcta tgatgatggc gaccggccct cagggatccc tgagcgattc     420

tctggctcca actctgggaa cacggccacc ctgaccatca gcagggtcga ggccggggat     480

gaggccgact attattgtca ggtgtgggat actggtagtg atcccgtggt attcggcgga     540

gggaccaagc tgaccgtcct aggtcagccc aaggctgccc cctcggtcac tctgttcccg     600

ccctcctctg aggagcttca agccaacaag gccacactgg tgtgtctcat aagtgacttc     660

tacccgggag ccgtgacagt ggcctggaag gcagatagca gccccgtcaa ggcgggagtg     720

gagaccacca caccctccaa acaaagcaac aacaagtacg cggccagcag ctatctgagc     780

ctgacgcctg agcagtggaa gtcccacaga agctacagct gccaggtcac gcatgaaggg     840

agcaccgtgg agaagacagt ggcccctaca gaatgttcat aggaattcat tgatcataat     900

cagccatacc acatttgtag aggttttact tgctttaaaa aacctcccac acctccccct 960

gaacctgaaa cataaaatga atgcaattgt tgttgttaac ttgtttattg cagcttataa 1020

tggttacaaa taaagcaata gcatcacaaa tttcacaaat aaagcatttt tttc 1074

<210> 2
<211> 1074
<212> DNA
<213> Artificial Sequence

<220>
<223> Complementary strand of CAT-354 light chain DNA sequence

<400> 2
agtctagcgg acctctgcgg taggtgcgac aaaactggag gtatcttctg tggccctggc 60

taggtcggag gcgccggccc ttgccacgta accttgcgcc taaggggcac ggttctcact 120

gagtggcagg aactgtgctt cgataagctt gatgtcaatg actcgtgtgt cctggagtgg 180

taccctacct cgacatagta ggagaagaac catcgttgtc gatgtccaca ggtgaggagg 240

atacacgact gagtcggtgg gagccacagt caccggggtc ctttctgccg gtcctaatgg 300

acaccccctt tgtagtaacc ttcatttgaa catgtgacca tggtcgtctt cggtccggtc 360

cggggacacg accagtagat actactaccg ctggccggga gtccctaggg actcgctaag 420

agaccgaggt tgagaccctt gtgccggtgg gactggtagt cgtcccagct ccggccccta 480

ctccggctga taataacagt ccacacccta tgaccatcac tagggcacca taagccgcct 540

ccctggttcg actggcagga tccagtcggg ttccgacggg ggagccagtg agacaagggc 600

gggaggagac tcctcgaagt tcggttgttc cggtgtgacc acacagagta ttcactgaag 660

atgggccctc ggcactgtca ccggaccttc cgtctatcgt cggggcagtt ccgccctcac 720

ctctggtggt gtgggaggtt tgtttcgttg ttgttcatgc gccggtcgtc gatagactcg 780

gactgcggac tcgtcacctt cagggtgtct tcgatgtcga cggtccagtg cgtacttccc 840

tcgtggcacc tcttctgtca ccggggatgt cttacaagta tccttaagta actagtatta 900

gtcggtatgg tgtaaacatc tccaaaatga acgaaatttt ttggagggtg tggaggggga 960

cttggacttt gtattttact tacgttaaca acaacaattg aacaaataac gtcgaatatt 1020

accaatgttt atttcgttat cgtagtgttt aaagtgttta tttcgtaaaa aaag 1074

<210> 3
<211> 233
<212> PRT
<213> Artificial Sequence

<220>
<223> CAT-354 light chain

<400> 3

Met Gly Trp Ser Cys Ile Ile Leu Phe Leu Val Ala Thr Ala Thr Gly
1               5                   10                  15

Val His Ser Ser Tyr Val Leu Thr Gln Pro Pro Ser Val Ser Val Ala
            20                  25                  30

Pro Gly Lys Thr Ala Arg Ile Thr Cys Gly Gly Asn Ile Ile Gly Ser
        35                  40                  45

Lys Leu Val His Trp Tyr Gln Gln Lys Pro Gly Gln Ala Pro Val Leu
    50                  55                  60

Val Ile Tyr Asp Asp Gly Asp Arg Pro Ser Gly Ile Pro Glu Arg Phe
65                  70                  75                  80

Ser Gly Ser Asn Ser Gly Asn Thr Ala Thr Leu Thr Ile Ser Arg Val
                85                  90                  95

Glu Ala Gly Asp Glu Ala Asp Tyr Tyr Cys Gln Val Trp Asp Thr Gly
            100                 105                 110

Ser Asp Pro Val Val Phe Gly Gly Gly Thr Lys Leu Thr Val Leu Gly
        115                 120                 125

Gln Pro Lys Ala Ala Pro Ser Val Thr Leu Phe Pro Pro Ser Ser Glu
    130                 135                 140

Glu Leu Gln Ala Asn Lys Ala Thr Leu Val Cys Leu Ile Ser Asp Phe
145                 150                 155                 160

Tyr Pro Gly Ala Val Thr Val Ala Trp Lys Ala Asp Ser Ser Pro Val
                165                 170                 175

Lys Ala Gly Val Glu Thr Thr Thr Pro Ser Lys Gln Ser Asn Asn Lys
            180                 185                 190

Tyr Ala Ala Ser Ser Tyr Leu Ser Leu Thr Pro Glu Gln Trp Lys Ser
        195                 200                 205

His Arg Ser Tyr Ser Cys Gln Val Thr His Glu Gly Ser Thr Val Glu
    210                 215                 220

Lys Thr Val Ala Pro Thr Glu Cys Ser
225                 230

<210>  4
<211>  1937
<212>  DNA

<213>  Artificial Sequence

<220>
<223>  Coding sequence for CAT-354 heavy chain DNA sequence

<400>  4
tcagatcgcc tggagacgcc atccacgctg ttttgacctc catagaagac accgggaccg      60

atccagcctc cgcggccggg aacggtgcat tggaacgcgg attccccgtg ccaagagtga     120

ctcaccgtcc ttgacacgaa gcttactgag cacacaggac ctcaccatgg gatggagctg     180

tatcatcctc ttcttggtag caacagctac aggtgtccac tcccaagtgc agctggtgca     240

gtctggggct gaggtgaaga agcctggggc ctcagtgaag gtctcctgca aggcttctgg     300

ttacaccttt acaaattatg gtctcagctg ggtgcgacag gcccctggac aagggcttga     360

gtggatggga tggatcagcg ctaataatgg cgacacaaat tatggacagg aattccaggg     420

cagagtcacc atgaccacag atacatccac gagcacagcc tacatggagt tgaggagcct     480

gagatctgac gacacggccg tttattactg tgcgagagac tccagcagca gctgggctcg     540

ctggtttttc gatctctggg gccgggggac actggtcacc gtctcgagtg cttccaccaa     600

gggcccatcc gtcttccccc tggcgccctg ctccaggagc acctccgaga gcacagccgc     660

cctgggctgc ctggtcaagg actacttccc cgaaccggtg acggtgtcgt ggaactcagg     720

cgccctgacc agcggcgtgc acaccttccc ggctgtccta cagtcctcag gactctactc     780

cctcagcagc gtggtgaccg tgccctccag cagcttgggc acgaagacct acacctgcaa     840

cgtagatcac aagcccagca acaccaaggt cgacaagaga gttgagtcca aatatggtcc     900

cccatgccca tcatgcccag cacctgagtt cctgggggga ccatcagtct tcctgttccc     960

cccaaaaccc aaggacactc tcatgatctc ccggacccct gaggtcacgt gcgtggtggt    1020

ggacgtgagc caggaagacc ccgaggtcca gttcaactgg tacgtggatg gcgtggaggt    1080

gcataatgcc aagacaaagc cgcgggagga gcagttcaac agcacgtacc gtgtggtcag    1140

cgtcctcacc gtcctgcacc aggactggct gaacggcaag gagtacaagt gcaaggtctc    1200

caacaaaggc ctcccgtcct ccatcgagaa aaccatctcc aaagccaaag ggcagccccg    1260

agagccacag gtgtacaccc tgcccccatc ccaggaggag atgaccaaga accaggtcag    1320

cctgacctgc ctggtcaaag gcttctaccc cagcgacatc gccgtggagt gggagagcaa    1380

tgggcagccg gagaacaact acaagaccac gcctcccgtg ctggactccg acggctcctt    1440

cttcctctac agcaggctaa ccgtggacaa gagcaggtgg caggagggga atgtcttctc    1500

atgctccgtg atgcatgagg ctctgcacaa ccactacaca cagaagagcc tctccctgtc    1560

tctgggtaaa tgagtgccag ggccggcaag cccccgctcc ccgggctctc ggggtcgcgc    1620

gaggatgctt ggcacgtacc ccgtctacat acttcccagg cacccagcat ggaaataaag    1680

cacccaccac tgccctggct cgaattcatt gatcataatc agccatacca catttgtaga    1740

ggttttactt gctttaaaaa acctcccaca cctccccctg aacctgaaac ataaaatgaa 1800

tgcaattgtt gttgttaact tgtttattgc agcttataat ggttacaaat aaagcaatag 1860

catcacaaat ttcacaaata aagcattttt ttcactgcat tctagttgtg gtttgtccaa 1920

actcatcaat gtatctt 1937

<210> 5
<211> 1937
<212> DNA
<213> Artificial Sequence

<220>
<223> Complementary strand for CAT-354 heavy chain DNA sequence

<400> 5
agtctagcgg acctctgcgg taggtgcgac aaaactggag gtatcttctg tggccctggc 60

taggtcggag gcgccggccc ttgccacgta accttgcgcc taaggggcac ggttctcact 120

gagtggcagg aactgtgctt cgaatgactc gtgtgtcctg gagtggtacc ctacctcgac 180

atagtaggag aagaaccatc gttgtcgatg tccacaggtg agggttcacg tcgaccacgt 240

cagaccccga ctccacttct tcggaccccg gagtcacttc cagaggacgt tccgaagacc 300

aatgtggaaa tgtttaatac cagagtcgac ccacgctgtc cggggacctg ttcccgaact 360

cacctaccct acctagtcgc gattattacc gctgtgttta atacctgtcc ttaaggtccc 420

gtctcagtgg tactggtgtc tatgtaggtg ctcgtgtcgg atgtacctca actcctcgga 480

ctctagactg ctgtgccggc aaataatgac acgctctctg aggtcgtcgt cgacccgagc 540

gaccaaaaag ctagagaccc cggccccctg tgaccagtgg cagagctcac gaaggtggtt 600

cccgggtagg cagaaggggg accgcgggac gaggtcctcg tggaggctct cgtgtcggcg 660

ggacccgacg gaccagttcc tgatgaaggg gcttggccac tgccacagca ccttgagtcc 720

gcgggactgg tcgccgcacg tgtggaaggg ccgacaggat gtcaggagtc ctgagatgag 780

ggagtcgtcg caccactggc acgggaggtc gtcgaacccg tgcttctgga tgtggacgtt 840

gcatctagtg ttcgggtcgt tgtggttcca gctgttctct caactcaggt ttataccagg 900

gggtacgggt agtacgggtc gtggactcaa ggacccccct ggtagtcaga aggacaaggg 960

gggttttggg ttcctgtgag agtactagag ggcctgggga ctccagtgca cgcaccacca 1020

cctgcactcg gtccttctgg ggctccaggt caagttgacc atgcacctac cgcacctcca 1080

cgtattacgg ttctgtttcg gcgccctcct cgtcaagttg tcgtgcatgg cacaccagtc 1140

gcaggagtgg caggacgtgg tcctgaccga cttgccgttc ctcatgttca cgttccagag 1200

gttgtttccg gagggcagga ggtagctctt ttggtagagg tttcggtttc ccgtcggggc 1260

tctcggtgtc cacatgtggg acgggggtag ggtcctcctc tactggttct tggtccagtc 1320

ggactggacg gaccagtttc cgaagatggg gtcgctgtag cggcacctca ccctctcgtt 1380

acccgtcggc ctcttgttga tgttctggtg cggagggcac gacctgaggc tgccgaggaa 1440

gaaggagatg tcgtccgatt ggcacctgtt ctcgtccacc gtcctcccct tacagaagag 1500

tacgaggcac tacgtactcc gagacgtgtt ggtgatgtgt gtcttctcgg agagggacag 1560

agacccattt actcacggtc ccggccgttc gggggcgagg ggcccgagag ccccagcgcg 1620

ctcctacgaa ccgtgcatgg ggcagatgta tgaagggtcc gtgggtcgta cctttatttc 1680

gtgggtggtg acgggaccga gcttaagtaa ctagtattag tcggtatggt gtaaacatct 1740

ccaaaatgaa cgaaattttt tggagggtgt ggagggggac ttggactttg tattttactt 1800

acgttaacaa caacaattga acaaataacg tcgaatatta ccaatgttta tttcgttatc 1860

gtagtgttta aagtgtttat ttcgtaaaaa aagtgacgta agatcaacac caaacaggtt 1920

tgagtagtta catagaa 1937

<210> 6
<211> 468
<212> PRT
<213> Artificial Sequence

<220>
<223> CAT-354 heavy chain

<400> 6

Met Gly Trp Ser Cys Ile Ile Leu Phe Leu Val Ala Thr Ala Thr Gly
1               5                   10                  15

Val His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Asn Tyr Gly Leu Ser Trp Val Arg Gln Ala Pro Gly Gln Gly Leu
    50                  55                  60

Glu Trp Met Gly Trp Ile Ser Ala Asn Asn Gly Asp Thr Asn Tyr Gly
65                  70                  75                  80

Gln Glu Phe Gln Gly Arg Val Thr Met Thr Thr Asp Thr Ser Thr Ser
                85                  90                  95

Thr Ala Tyr Met Glu Leu Arg Ser Leu Arg Ser Asp Asp Thr Ala Val
            100                 105                 110

Tyr Tyr Cys Ala Arg Asp Ser Ser Ser Ser Trp Ala Arg Trp Phe Phe
        115                 120                 125

Asp Leu Trp Gly Arg Gly Thr Leu Val Thr Val Ser Ser Ala Ser Thr
    130                 135                 140

Lys Gly Pro Ser Val Phe Pro Leu Ala Pro Cys Ser Arg Ser Thr Ser
145                 150                 155                 160

Glu Ser Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Lys Thr Tyr Thr Cys
    210                 215                 220

Asn Val Asp His Lys Pro Ser Asn Thr Lys Val Asp Lys Arg Val Glu
225                 230                 235                 240

Ser Lys Tyr Gly Pro Pro Cys Pro Ser Cys Pro Ala Pro Glu Phe Leu
                245                 250                 255

Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser
        275                 280                 285

Gln Glu Asp Pro Glu Val Gln Phe Asn Trp Tyr Val Asp Gly Val Glu
    290                 295                 300

Val His Asn Ala Lys Thr Lys Pro Arg Glu Glu Gln Phe Asn Ser Thr
305                 310                 315                 320

Tyr Arg Val Val Ser Val Leu Thr Val Leu His Gln Asp Trp Leu Asn
                325                 330                 335

Gly Lys Glu Tyr Lys Cys Lys Val Ser Asn Lys Gly Leu Pro Ser Ser
            340                 345                 350

Ile Glu Lys Thr Ile Ser Lys Ala Lys Gly Gln Pro Arg Glu Pro Gln
        355                 360                 365

Val Tyr Thr Leu Pro Pro Ser Gln Glu Glu Met Thr Lys Asn Gln Val

370                 375                 380

Ser Leu Thr Cys Leu Val Lys Gly Phe Tyr Pro Ser Asp Ile Ala Val
385                 390                 395                 400

Glu Trp Glu Ser Asn Gly Gln Pro Glu Asn Asn Tyr Lys Thr Thr Pro
                405                 410                 415

Pro Val Leu Asp Ser Asp Gly Ser Phe Phe Leu Tyr Ser Arg Leu Thr
            420                 425                 430

Val Asp Lys Ser Arg Trp Gln Glu Gly Asn Val Phe Ser Cys Ser Val
        435                 440                 445

Met His Glu Ala Leu His Asn His Tyr Thr Gln Lys Ser Leu Ser Leu
    450                 455                 460

Ser Leu Gly Lys
465

<210>  7
<211>  456
<212>  PRT
<213>  Artificial Sequence

<220>
<223>  MEDI8897 heavy chain

<400>  7

Gln Val Gln Leu Val Gln Ser Gly Ala Glu Val Lys Lys Pro Gly Ser
1               5                   10                  15

Ser Val Met Val Ser Cys Gln Ala Ser Gly Gly Leu Leu Glu Asp Tyr
            20                  25                  30

Ile Ile Asn Trp Val Arg Gln Ala Pro Gly Gln Gly Pro Glu Trp Met
        35                  40                  45

Gly Gly Ile Ile Pro Val Leu Gly Thr Val His Tyr Gly Pro Lys Phe
    50                  55                  60

Gln Gly Arg Val Thr Ile Thr Ala Asp Glu Ser Thr Asp Thr Ala Tyr
65                  70                  75                  80

Met Glu Leu Ser Ser Leu Arg Ser Glu Asp Thr Ala Met Tyr Tyr Cys
                85                  90                  95

Ala Thr Glu Thr Ala Leu Val Val Ser Glu Thr Tyr Leu Pro His Tyr
            100                 105                 110

Phe Asp Asn Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Arg Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Tyr Ile Thr Arg Glu Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Glu Glu Met Thr

355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys
    450                 455

<210>  8
<211>  1368
<212>  DNA
<213>  Artificial Sequence

<220>
<223>  MEDI8897 heavy chain

<400>  8
caagtgcagc tggtgcagtc tggcgccgaa gtgaagaaac ccggctcctc cgtgatggtg      60

tcctgccagg cttctggcgg cctgctggaa gattacatca tcaactgggt gcgacaggcc     120

ccaggccagg gacctgaatg gatgggcgga atcatccccg tgctgggcac cgtgcactac     180

ggccctaagt tccagggcag agtgaccatc accgccgacg agtctaccga caccgcctac     240

atggaactgt cctccctgcg gagcgaggac accgccatgt actactgcgc caccgagaca     300

gccctggtgg tgtccgagac atacctgccc cactacttcg acaactgggg ccagggaacc     360

ctcgtgaccg tctcctcagc ctccaccaag ggcccatcgg tcttccccct ggcaccctcc     420

tccaagtcca cctccggcgg caccgccgct ctgggctgcc tggtgaagga ctacttccct     480

gagcctgtga ccgtgtcctg gaactctggc gccctgacct ctggcgtgca caccttccct     540

gccgtgctgc agtcctccgg cctgtactcc ctgtcctccg tggtgacagt gccttcctcc     600

tccctgggca cccagaccta catctgcaac gtgaaccaca agcccagcaa caccaaggtg     660

gacaagagag ttgagcccaa atcttgtgac aaaactcaca catgcccacc gtgcccagca     720

cctgaactcc tggggggacc gtcagtcttt ctgttccctc ctaagcctaa ggacaccctg     780

tacatcaccc gggagcctga agtgacctgc gtggtggtgg atgtgtccca cgaggaccct     840

gaggtgaagt tcaattggta cgtggacggc gtggaggtgc acaacgccaa gaccaagcct 900

cgggaggagc agtacaactc cacctaccgg gtggtgtctg tgctgaccgt gctgcaccag 960

gactggctga acggcaaaga atacaagtgc aaagtctcca acaaggccct gcctgccccc 1020

atcgagaaaa ccatctccaa agccaaaggg cagccccgag aaccacaggt gtacaccctg 1080

cctccctccc gcgaggagat gaccaagaac caggtgtccc tgacctgtct ggtgaagggc 1140

ttctaccctt ccgatatcgc cgtggagtgg gagtccaacg gccagcctga gaacaactac 1200

aagaccaccc ctcctgtgct ggactccgac ggctccttct tcctgtactc caagctgacc 1260

gtggacaagt cccggtggca gcagggcaac gtgttctcct gctccgtgat gcacgaggct 1320

ctgcacaacc actacaccca gaaaagcctc tccctgtctc cgggtaaa 1368

<210> 9
<211> 214
<212> PRT
<213> Artificial Sequence

<220>
<223> MEDI8897 light chain

<400> 9

Asp Ile Gln Met Thr Gln Ser Pro Ser Ser Leu Ser Ala Ala Val Gly
1               5                   10                  15

Asp Arg Val Thr Ile Thr Cys Gln Ala Ser Gln Asp Ile Val Asn Tyr
            20                  25                  30

Leu Asn Trp Tyr Gln Gln Lys Pro Gly Lys Ala Pro Lys Leu Leu Ile
        35                  40                  45

Tyr Val Ala Ser Asn Leu Glu Thr Gly Val Pro Ser Arg Phe Ser Gly
    50                  55                  60

Ser Gly Ser Gly Thr Asp Phe Ser Leu Thr Ile Ser Ser Leu Gln Pro
65                  70                  75                  80

Glu Asp Val Ala Thr Tyr Tyr Cys Gln Gln Tyr Asp Asn Leu Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala

130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  10
<211>  642
<212>  DNA
<213>  Artificial Sequence

<220>
<223>  MEDI8897 light chain

<400>  10
gacatccaga tgacccagtc cccctcctct ctgtctgctg ccgtgggcga cagagtgacc      60

atcacctgtc aggcctccca ggacatcgtg aactacctga actggtatca gcagaagccc     120

ggcaaggccc ccaagctgct gatctacgtg gcctccaacc tggaaaccgg cgtgccctcc     180

agattctccg gctctggctc tggcaccgac ttcagcctga ccatctccag cctgcagcct     240

gaggacgtgg ccacctacta ctgccagcag tacgacaacc tgcccctgac ctttggcgga     300

ggcaccaagg tggagatcaa acgaactgtg gctgcaccat ctgtcttcat cttccccccc     360

agcgacgagc agctgaagag cggcaccgcc tccgtggtgt gcctgctgaa caacttctac     420

ccccgcgagg ccaaggtgca gtggaaggtg gacaacgccc tgcagtccgg caacagccag     480

gagagcgtca ccgagcagga cagcaaggac tccacctaca gcctgagcag caccctgacc     540

ctgagcaagg ccgactacga gaagcacaag gtgtacgcct gcgaggtgac ccaccagggc     600

ctgtccagcc ccgtgaccaa gagcttcaac aggggcgagt gc                        642

## Description

### FIELD OF THE INVENTION

The invention is concerned with an antibody formulation, in particular, a monoclonal antibody formulation and uses thereof. The invention is particularly concerned with providing improved colloidal stability in an antibody formulation.

### BACKGROUND OF THE INVENTION

As a result of the isoelectric point (pI) of a number of monoclonal antibodies being in the preferred pharmaceutical pH formulation range for proteins (pH 5.5 to pH 7.5), these molecules present unique formulation challenges.

Colloidal instability at a molecule's pI is due to a lack of an electrostatic charge on the molecule, which allows closer protein-protein interactions (so-called "self-association") that lead to physical instabilities. For this reason, the pH of a protein formulation is typically selected to be at least 1 pH unit away from the protein pI. This aims to provide colloidal stability and thus prevent physical instabilities, such as aggregation, precipitation, opalescence, phase separation and/or particle formation.

According to the '1 pH unit away' rule, antibodies having a low or neutral pI *e.g.* a pI of pH 5.5 to pH 7.5 thus should be formulated into a formulation with a pH outside the range of 5.5 to 7.5. However, outside this range, additional instabilities can be observed. At more acidic pH, an increased rate of fragmentation, reduced conformational stability and increased aggregation can be observed. At more basic pH, the potential for increased oxidation, deamidation and fragmentation and incompatibility with glass containers are present.

The above instabilities are particularly problematic in such antibody formulations where the antibody is present at a commercially desirable concentration *e.g.* 50mg/ml and above.

Therefore, there exists a need to provide an improved formulation for an antibody having a low or neutral pI. In particular, there exists a need to provide a stable formulation for an antibody having a low or neutral pI and, particularly such a formulation having a commercially desirable antibody concentration.

### BRIEF SUMMARY OF THE INVENTION

The present invention provides a new antibody formulation, in particular a new monoclonal antibody formulation. In particular, the present formulation provides a means for improving colloidal stability for antibodies having a low or neutral pI. The present invention thus provides an alternative to the '1 pH away' rule for providing colloidal stability. The present invention thus allows antibodies having a low or neutral pI to be formulated within 1 pH unit of the antibody pI. Thus, the present invention enables such antibodies to be formulated within a pH range of 5.5 to 7.5 and at a commercially useful concentration, whilst substantially avoiding the instabilities associated with more acidic or more basic pHs.

The invention provides a formulation comprising:
i. a monoclonal antibody; and
ii. an ionic excipient;
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5.

The invention thus further provides a formulation comprising:
i. a monoclonal antibody; and
ii. an ionic excipient;
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient.

The formulations of the present invention are particularly useful for antibodies having a low or neutral pI, for example in the range of pH 5.5 to pH 7.5. The invention provides a formulation comprising:
i. a monoclonal antibody having a low or neutral pI (e.g. 5.5 to 7.5); and
ii. an ionic excipient;
wherein the monoclonal antibody is present at a concentration of about 50mg/ml or greater (e.g. about 50 mg/ml to about 200 mg/ml) and the ionic excipient is present at a concentration of about 50 to about 150mM and the formulation has a pH of 5.5 to 7.5.

The invention thus further provides a formulation comprising:
i. a monoclonal antibody having a low or neutral pI (e.g. 5.5 to 7.5); and
ii. an ionic excipient;
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody formulated in the same formulation but without an ionic excipient.

In one embodiment, the monoclonal antibody has a pI in the range of 5.5 to 7.5. In one embodiment, the monoclonal antibody has a pI in the range of 6.0 to 7.5. In one embodiment, the monoclonal antibody has a pI in the range of 6.3 to 7.5. In one embodiment, the monoclonal antibody has a pI in the range of 6.4 to 7.5. Without wishing to be bound by theory, a low to neutral pI can occur for proteins where there is a either a net balance of oppositely charged (positive amine groups and negative carboxylate groups) amino acid side chains on the protein or different domains have overall opposite charge, within a pH range of 5.5 to 7.5. Again, without wishing to be bound by theory, it is possible that the ionic excipient in the formulation of the invention shields these opposing and attractive charges, thus colloidally stabilizing proteins having a pI within this range. The present invention thus provides use of an ionic excipient in an antibody formulation for the purpose of changing the charge state or distribution of the antibody in the formulation. The present invention further provides use of an ionic excipient in an antibody formulation for the purpose of colloidally stabilizing the antibody in the formulation.

In one embodiment, the monoclonal antibody is present in the formulations described herein at a concentration of about 75 mg/ml or greater (e.g. about 75 mg/ml to about 200 mg/ml). In one embodiment, the monoclonal antibody is present in the formulations described herein at a concentration of about 100 mg/ml or greater (e.g. about 100 mg/ml to about 200 mg/ml). In one embodiment, the monoclonal antibody is present in the formulations described herein at a concentration of about 100 mg/ml to about 165 mg/ml. In one embodiment, the monoclonal antibody is present at a concentration of about 100mg/ml.

In one embodiment, the ionic excipient is present at a concentration of about 75 mM to about 100 mM. In one embodiment, the ionic excipient is present at a concentration of about 75 mM. In one embodiment, the ionic excipient is present at a concentration of about 80 mM.

In one embodiment, the monoclonal antibody is an IgG1 or IgG4 monoclonal antibody. Most preferably, the monoclonal antibody is an IgG4 monoclonal antibody. IgG4 antibodies typically have a low or neutral pI. The invention thus provides a formulation comprising:
i. an IgG4 monoclonal antibody; and
ii. an ionic excipient;
   wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5.

The invention thus further provides a formulation comprising:
i. an IgG4 monoclonal antibody; and
ii. an ionic excipient;
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient.

In one embodiment, the formulations described herein have a pH in the range of about pH 5.5 to about pH 6.5. In one embodiment, the formulations described herein have a pH in the range of about pH 5.7 to about pH 6.3. In one embodiment, the formulations described herein have a pH in the range of about pH 5.7 to about pH 6.1. Preferred formulations have a pH of about 5.8. Other preferred formulations have a pH of about 6.0.

In one embodiment, the ionic excipient is a charged amino acid. In one embodiment, the ionic excipient is lysine. In another embodiment, the ionic excipient is arginine.

In one embodiment, the ionic excipient is a salt. The invention thus provides a formulation comprising:
i. a monoclonal antibody as defined anywhere herein; and
ii. a salt;
wherein the monoclonal antibody is present at a concentration of about 50mg/ml or greater (e.g. about 50 mg/ml to about 200 mg/ml) and the salt is present at a concentration of about 50 to about 150 mM and the formulation has a pH of 5.5 to 7.5.

The invention thus further provides a formulation comprising:
i. a monoclonal antibody as defined anywhere herein; and
ii. a salt;
wherein the monoclonal antibody is present at a concentration of about 50mg/ml or greater (e.g. about 50 mg/ml to about 200 mg/ml) and the salt is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without a salt.

In one embodiment, the salt is present at a concentration of about 75 mM to about 100 mM. In one embodiment, the salt is present at a concentration of about 75 mM or about 80 mM.

In one embodiment, the salt is NaCl, for example at a concentration of about 75 mM to about 100 mM, suitably at a concentration of about 75 mM.

In one embodiment, the salt is arginine hydrochloride, for example at a concentration of about 75 mM to about 100 mM, suitably at a concentration of about 80 mM.

In one embodiment, the formulation further comprises a sugar. Amongst other known benefits, the presence of a sugar can improve tonicity of the formulation. This is desirable since preferred formulations are isotonic or near isotonic (for example, having an osmolality between 240 to 500 mOsm/kg. In one embodiment, the ionic excipient is a salt and the formulation further comprises a sugar.

In one embodiment, the formulation further comprises a sugar and the ionic excipient is present at a concentration in the range of about 75mM to about 150 mM. In one embodiment, the formulation further comprises a sugar and the ionic excipient is present at a concentration in the range of about 75mM to about 100mM. In one embodiment, the formulation further comprises a sugar, which is present at a concentration in the range of about 100mM to 140mM, and the ionic excipient is present at a concentration in the range of about 75mM to 100mM.

The invention thus provides a formulation comprising:
i. a monoclonal antibody as defined anywhere herein;
ii. an ionic excipient *(e.g.* a salt) as defined anywhere herein;
iii. a sugar as defined anywhere herein; and
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5. The ionic excipient preferably is present at a concentration in the range of about 75mM to about 150 mM, more preferably about 75mM to about 100 mM.

The invention thus further provides a formulation comprising:
i. a monoclonal antibody as defined anywhere herein; and
ii. an ionic excipient (*e.g.* a salt) as defined anywhere herein;
iii. a sugar as defined anywhere herein; and
wherein the monoclonal antibody is present at a concentration of about 50mg/ml or greater (e.g. about 50 mg/ml to about 200 mg/ml) and the ionic excipient is present at a concentration of about 50 of about 150 mM and the formulation has a pH of 5.5 to 7.5; and wherein the aggregation rate of the monoclonal antibody in the formulation is reduced compared to the aggregation rate of the same antibody in the same formulation but without an ionic excipient. The ionic excipient preferably is present at a concentration in the range of about 75mM to about 150 mM, more preferably about 75mM to about 100 mM.

In one embodiment, the sugar is trehalose. In another embodiment, the sugar is sucrose. For example, the sugar is found at a concentration of about 100 mM to about 140 mM, suitably at a concentration of about 120 mM.

In one embodiment, the formulation further comprises one or more buffers. In one embodiment, the one or more buffers is a buffer comprising histidine. In one embodiment, the one or more buffers are selected from a buffer comprising histidine succinate, histidine acetate, histidine citrate, histidine chloride or histidine sulfate. In one embodiment, the one or more buffers is histidine, histidine hydrochloride, or a combination thereof (histidine/ histidine hydrochloride). In one embodiment, the one or more buffers is L-histidine/ L-histidine hydrochloride monohydrate. For example, the buffer may be at a concentration of about 10 mM to about 50 mM, suitably at a concentration of about 30 mM. It will be understood that a buffer may, itself, be an ionic excipient. Thus, in one embodiment, the buffer is the ionic excipient. In this embodiment, the concentration of the buffer should be above 50 mM i.e. in line with the concentration of the ionic excipient disclosed herein. Put another way, in one embodiment, the ionic excipient also acts as a buffer in the formulation. In this embodiment, an additional buffer may or may not be present.

In one embodiment, the formulation further comprises a surfactant. In one embodiment, the surfactant is a polysorbate, including for example, polysorbate-80.

In one embodiment, the formulation further comprises a sugar and one or more buffers. In one embodiment, the ionic excipient is a salt and the formulation further comprises a sugar and one or more buffers.

In one embodiment, the formulation further comprises a surfactant, a sugar and one or more buffers. In one embodiment, the ionic excipient is a salt and the formulation further comprises a surfactant, a sugar and one or more buffers.

The invention thus provides a formulation comprising:
i. a monoclonal antibody as defined anywhere herein;
ii. an ionic excipient (*e.g.* a salt) as defined anywhere herein;
iii. a sugar as defined anywhere herein;
iv. one or more buffers as defined anywhere herein; and
v. optionally a surfactant as defined anywhere herein
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5.

The invention thus further provides a formulation comprising:
i. a monoclonal antibody as defined anywhere herein; and
ii. an ionic excipient (e.g. a salt) as defined anywhere herein;
iii. a sugar as defined anywhere herein;
iv. one or more buffers as defined anywhere herein; and
v. optionally a surfactant as defined anywhere herein
wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient..

The formulations described herein can also include one or more additional excipients, including for example, one or more sugars, salts, amino acids, polyols, chelating agents, emulsifiers and/or preservatives.

One preferred formulation provided by the invention comprises 150mg/ml antibody, 50mM sodium acetate/acetic acid, 106mM trehalose dehydrate, 70mM sodium chloride, 0.05% (w/v) Polysorbate 80, wherein the formulation has a pH of pH 5.8. The antibody in this formulation is preferably an anti-GM-GSF-Rα IgG4 antibody, more preferably an anti-GM-CSF-Ra IgG4 antibody having the VH and VL sequences of the CAM-3001 antibody as described herein. The antibody in this formulation is preferably an anti-GM-CSF-Ra IgG4 antibody having the same 6 CDRs as the CAM-3001 antibody as described herein.

One preferred formulation provided by the invention comprises 150mg/ml antibody, 50 mM Sodium Acetate, 85 mM sodium chloride and 0.01% Polysorbate 80, wherein the formulation has a pH of pH 5.5. The antibody in this formulation is preferably an anti-IL-13 IgG4 antibody, more preferably an anti-IL-13 IgG4 antibody having the VH and VL sequences of the CAT-354 antibody as described herein. The antibody in this formulation is preferably an anti-IL-13 IgG4 antibody having the same 6 CDRs as the CAT-354 antibody as described herein.

The formulations of the invention preferably arc pharmaceutical formulations.

The present invention provides a pharmaceutical formulation as described anywhere herein for use as a medicament.

The present invention provides a pharmaceutical formulation as described anywhere herein for use in the treatment of a disease.

The present invention provides a method of treating a disease in a subject comprising administering a pharmaceutical formulation as described anywhere herein to the subject. Also provided herein are methods of treating a subject by administering a therapeutically effective amount of a pharmaceutical formulation as described anywhere herein to the subject.

In one embodiment, the subject is a human.

The disease to be treated will be dependent on the particular antibody contained in the formulation. In one embodiment, the disease is cancer. The disease may be selected from the group consisting of diabetes, cardiovascular diseases, infectious disease, rheumatoid arthritis, vasculitis, giant cell arthritis, glomerular nephropathy, lupus nephritis, uveitis, atopic dermatitis, cirrhosis, psoriatic arthritis, chronic obstructive pulmonary disease, severe asthma, neutrophilic asthma, and myeloid leukemia.

The present invention provides a lyophilized cake capable of being reconstituted using only sterile water into a formulation as defined herein or a pharmaceutical formulation as defined herein. Also provided herein is a formulation capable of being lyophilised to form a lyophilized cake, wherein the lyophilized cake is capable of being reconstituted using only sterile water into a formulation as defined herein or a pharmaceutical formulation as defined herein.

### BRIEF DESCRIPTIONS OF THE DRAWINGS

FIG. 1 shows the conversion of an IgG4 monoclonal antibody to a half molecule.
FIG. 1-1 and FIG. 1-2 show sequences relating to the CAT-354 antibody. The V_{H} and V_{L} sequences are labelled and the 6 CDRs are underlined.
FIG. 2 shows the effect of salt on the aggregation rate of IgG4 molecules.
FIG. 3A shows the results of varying pH on k_{D}.
FIG. 3B shows the results of varying pH on Kd in the presence of 100 mM salt.
FIG. 4 shows the effect of salt on k_{D}. Note generally a reduction in colloidal stability at pH 5, but an increase in colloidal stability at pH 6 and pH 7.
FIG. 5 shows aggregation rate data at 40⁰C for formulations with or without 100 mM salt.
FIG. 6 shows change in aggregation rate with formulation pH for MEDI7814.
FIG. 7 shows the impact of ionic excipients on antibody aggregation rate at pH6 (MEDI7814).
FIG. 8 shows the MEDI8897 heavy chain nucleotide sequence and translation. CDRs are underlined, amino acid differences form allelic constant regions have been circled and division between the variable and constant regions marked by a '|').
FIG. 9 shows the MEDI8897 light chain nucleotide sequence and translation. CDRs are underlined and division between the variable and constant regions marked by a '|').
FIG. 10 shows MEDI8897 formulation stability over 3 month period at 5°C, 25°C and 40°C.
FIG. 11 shows the impact of ionic excipients on antibody aggregation rate at pH 6 (MEDI578).
FIG. 12 shows purity by size-exclusion chromatography.
FIG. 13 shows a reduction in subvisible particles in formulations containing ionic excipients under thermal stress conditions. Colloidal stability is improved.

### DETAILED DESCRIPTION OF THE INVENTION

Due to the fact that a number of monoclonal antibodies, and in particular IgG4 antibodies, possess a pI that is close to physiologic pH, *i.e.* the pH generally desired for human administration, difficulties in formulating these monoclonal antibodies occur. These difficulties can relate particularly to colloidal stability.

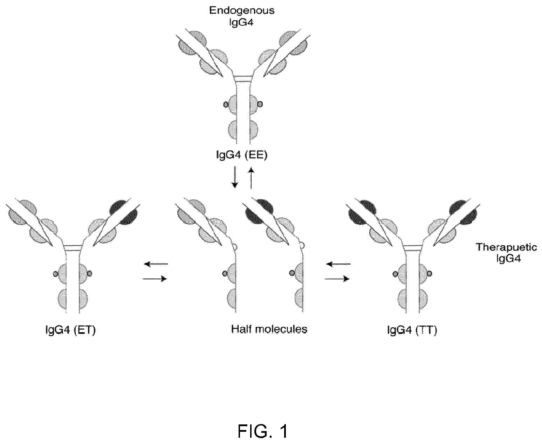

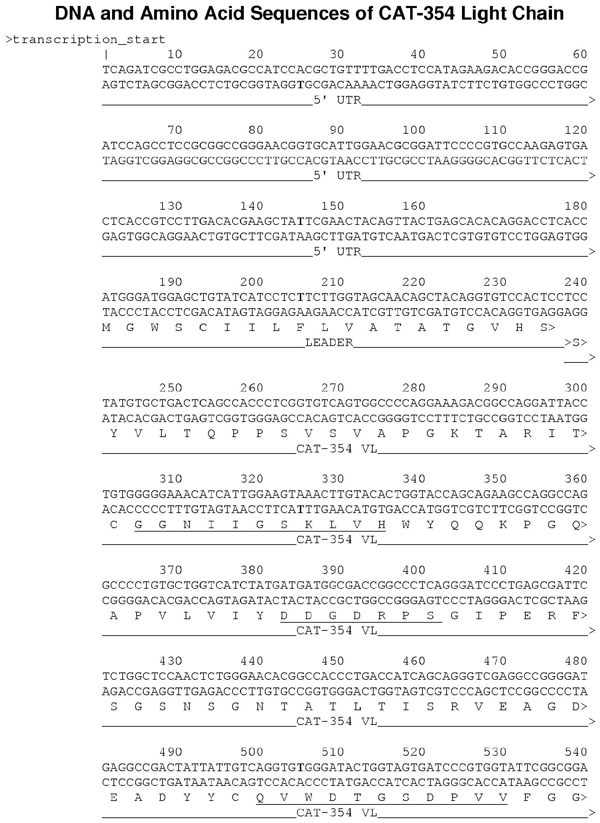

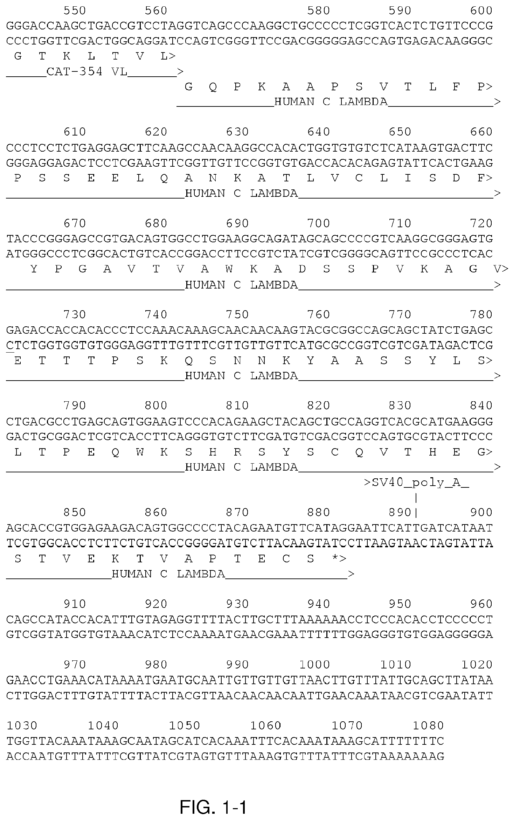

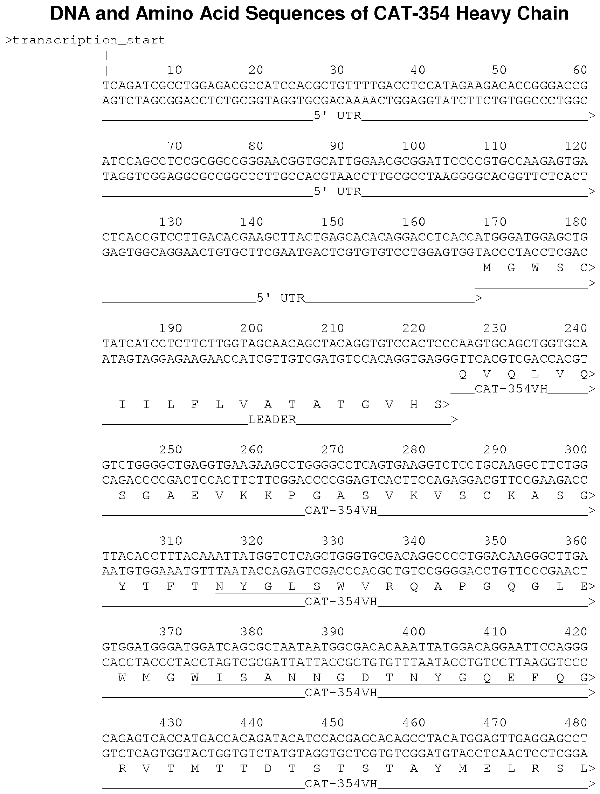

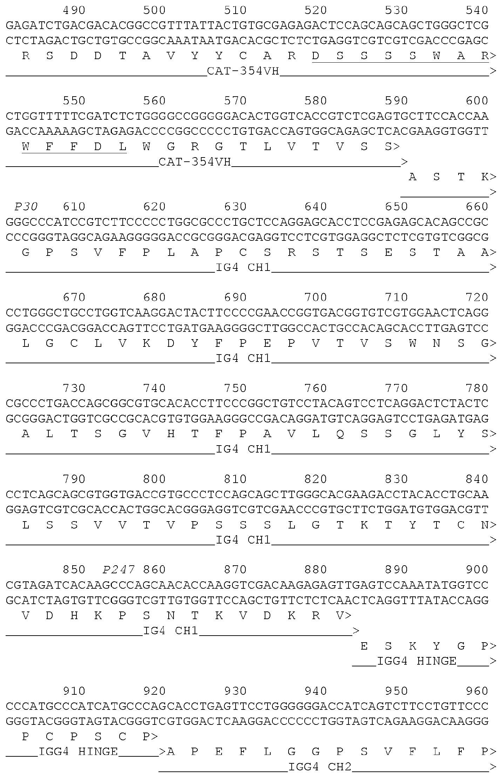

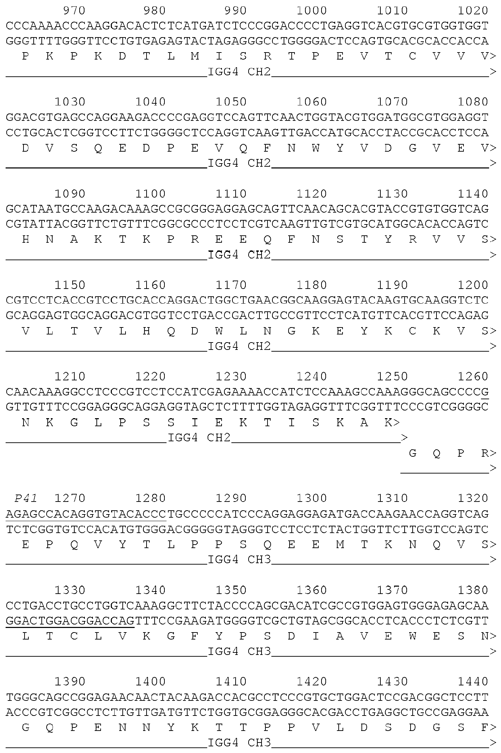

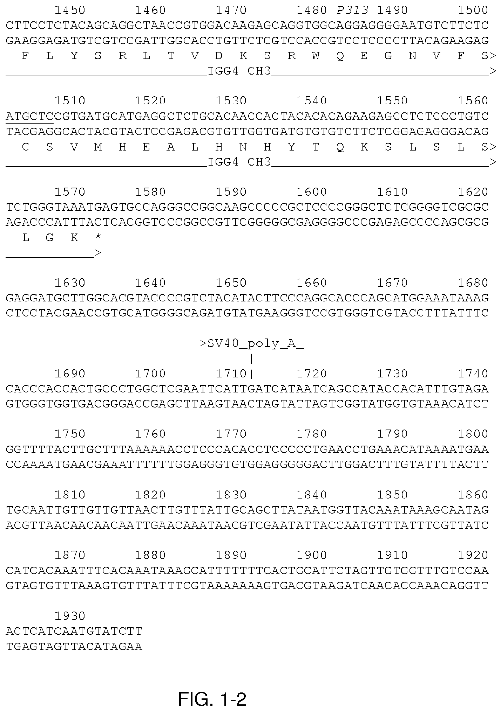

IgG4 monoclonal antibodies are characterized by a structure which prevents complement activation, and also allows for an *in vivo* half molecular exchange (one heavy and one light chain) between two IgG4 molecules, generating a new IgG4 with a bivalent reactivity. *See* FIG. 1. The IgG4 hinge is three amino acids shorter than the hinge of IgG1, and IgG4 has two cysteines that are available for the covalent interaction between the H-chains. [Aalberse and Schuurman, "lgG4 breaking the rules," Immunology 2002 105:9-19].

The present invention provides a new monoclonal antibody formulation, in particular a new IgG4 monoclonal antibody formulation and a new IgG1 monoclonal antibody formulation.

The invention provides a formulation comprising: (i) a monoclonal antibody; and (ii) an ionic excipient (*e.g*. a salt); wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5.

The invention further provides a formulation comprising: (i) a monoclonal antibody; and (ii) an ionic excipient (*e.g.* a salt); wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient.

Aggregation rate can be measured according to standard techniques as described herein. Surprisingly, formulations in accordance with the present invention have been shown to have good stability and to have decreased self-aggregation *e.g.* to exhibit ≤ 2.0 % aggregation when stored at room temperature for 3 months. The present invention thus provides the use of an ionic excipient in an antibody formulation for the purpose of increasing stability of the antibody in the formulation. The present invention further provides the use of an ionic excipient in an antibody formulation for the purpose of decreasing self-aggregation of the antibody in the formulation.

The formulations of the present invention are particularly useful for antibodies having a low or neutral pI, for example antibodies that have a pI in the range pH 5.5 to pH 7.5, pH 6.0 to pH 7.5, pH 6.3 to pH 7.5, pH 6.4 to pH 7.5, or pH 6.5 to pH 7.5. The pI of an antibody can be measured according to standard techniques, for example by capillary isoelectric focusing (cIEF). The invention thus provides a formulation comprising: (i) a monoclonal antibody having a low or neutral pI (e.g. 5.5 to 7.5); and (ii) an ionic cxcipient; wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5. The invention thus further provides a formulation comprising: (i) a monoclonal antibody having a low or neutral pI (e.g. 5.5 to 7.5); and (ii) an ionic excipient; wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient.

In one embodiment, the monoclonal antibodies have a pI in the range of pH 6.4 to pH 7.5. In another embodiment, the monoclonal antibody described herein has a pI in the range of about pH 5.5 to about pH 6.0, about pH 5.7 to about pH 6.0, or about pH 5.5, about pH 5.6, about pH 5.7, about pH 5.8, about pH 5.9, about pH 6.0, about pH 6.1, about pH 6.2, about pH 6.3, about pH 6.4, or about pH 6.5. In embodiments, the pI of the monoclonal antibodies provided herein is 5.7 to 6.0, more suitably a pI of about 5.8 or 6.0.

In one embodiment, the monoclonal antibody is an IgG1 or IgG4 monoclonal antibody. Most preferably, the monoclonal antibody is an IgG4 monoclonal antibody. The invention thus provides a formulation comprising: (i) an IgG4 monoclonal antibody having a low or neutral pI (e.g. 5.5 to 7.5); and (ii) an ionic excipient; wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5. The invention thus further provides a formulation comprising: (i) an IgG4 monoclonal antibody having a low or neutral pI (e.g. 5.5 to 7.5); and (ii) an ionic excipient; wherein the monoclonal antibody is present at a concentration of about 50mg/ml or greater (e.g. about 50 mg/ml to about 200 mg/ml) and the ionic excipient is present at a concentration of about 50 to about 150 mM and the formulation has a pH of 5.5 to 7.5; and wherein the aggregation rate of the monoclonal antibody in the formulation is reduced compared to the aggregation rate of the same antibody in the same formulation but without an ionic excipient.

In one embodiment, the monoclonal antibody is an anti-GM-CSF-Ra monoclonal antibody, an anti-IL-13 monoclonal antibody, an anti-RSV monoclonal antibody, or an anti-C5/C5a monoclonal antibody. In exemplary embodiments, the monoclonal antibody is an IgG4 monoclonal antibody, for example, an anti-GM-CSF-Ra monoclonal antibody. In one embodiment, the monoclonal antibody is is CAM-3001. In one embodiment, the monoclonal antibody is MEDI8897.

Monoclonal antibodies include antibody functional parts, e.g., antibodies or antigen-binding fragments, variants, or derivatives thereof. Monoclonal antibodies further include, but are not limited to, human, humanized, or chimeric antibodies, single chain antibodies, bispecific antibodies, epitope-binding fragments, e.g., Fab, Fab' and F(ab')2, Fd, Fvs, single-chain Fvs (scFv), single-chain antibodies, disulfide-linked Fvs (sdFv), fragments comprising either a VL or VH domain, fragments produced by a Fab expression library. ScFv molecules are known in the art and are described, e.g., in US patent 5,892,019. Immunoglobulin or antibody molecules encompassed by this disclosure can be of any type (e.g., IgG, IgE, IgM, IgD, IgA, and IgY), class (e.g., IgG1, IgG2, IgG3, IgG4, IgA1 and IgA2) or subclass of immunoglobulin molecule. In a preferred embodiment, the monoclonal antibody is an IgG1 or IgG4 monoclonal antibody.

### Antibody Concentration

Suitably, the monoclonal antibody is present in the formulations described herein at a commercially desirable concentration e.g. of about 50 mg/ml to about 300 mg/ml, about 50 mg/ml to about 200 mg/ml, about 100 mg/ml to about 200 mg/ml, about 100 mg/ml to about 165 mg/ml, about 100 mg/ml to about 150 mg/ml, or about 50 mg/ml, about 75 mg/ml, about 100 mg/ml, about 105 mg/ml, about 110 mg/ml, about 115 mg/ml, about 120 mg/ml, about 125 mg/ml, about 130 mg/ml, about 135 mg/ml, about 140 mg/ml, about 145 mg/ml, about 150 mg/ml, about 155 mg/ml, about 160 mg/ml, about 165 mg/ml, about 170 mg/ml, about 175 mg/ml, about 180 mg/ml, about 185 mg/ml, about 190 mg/ml, about 195 mg/ml, or about 200 mg/ml, including values and ranges within these ranges.

Suitably, the monoclonal antibody is present in the formulations described herein at a concentration of about 100 mg/ml to about 165 mg/ml. Suitably, the monoclonal antibody is present in the formulations described herein at a concentration of about 100 mg/ml.

### pH

Suitably, the formulations described herein have a pH in the range of about pH 5.5 to about pH 7.5 in order to provide near optimal or optimal chemical stability (hydrolysis, deamidation, isomerization). In one embodiment, the formulations described herein have a pH in the range of about pH 5.7 to about pH 6.3. In one embodiment, the formulations described herein have a pH in the range of about 5.5 to about 6.5. In one embodiment, the formulations described herein have a pH in the range of about pH 5.7 to about pH 6.1. Preferred formulations have a pH of about 5.8. Other preferred formulations have a pH of about 6.0.

Suitably, the formulations described herein have a pH in the range of about pH 5.5 to about pH 6.0, about pH 5.7 to about pH 6.0, or about pH 5.5, about pH 5.6, about pH 5.7, about pH 5.8, about pH 5.9, about pH 6.0, about pH 6.1, about pH 6.2, about pH 6.3, about pH 6.4, or about pH 6.5. In embodiments, the pH of the formulations provided herein is 5.7 to 6.0, more suitably the formulations have a pH of about 5.8 or 6.0.

A formulation pH close to about pH 7.4 also can be desirable for injection site tolerability.

### Ionic Excipient

Exemplary ionic excipients for use in the formulations include salts and charged amino acids. The ionic excipient might comprise a combination of a salt and charged amino acid.

Exemplary charged amino acids include arginine and lysine.

Exemplary salts include salts of charged amino acids, for example, succinate, acetate, and sulfate salts of arginine and lysine.

Further exemplary salts arc those described herein including, but not limited to, sodium chloride, as well as other salts with sodium, potassium, calcium, magnesium and the like, such as chlorides, carbonates, sulphates, acetates, gluconates, lactates, malates, and other auxiliaries and the like which are customary in the field of parenteral administration.

Suitably the salt is selected from sodium chloride (NaCl), lysine hydrochloride and arginine hydrochloride. In one embodiment, the salt is NaCl. In another embodiment, the salt is arginine hydrochloride. In another embodiment, the salt is lysine hydrochloride.

The concentration of the ionic excipient, suitably salt, in the pharmaceutical formulations described herein is generally in the range of about 50 mM to about 150 mM, more suitably about 50 mM to about 100 mM, about 60 mM to about 80 mM, or about 50 mM, about 55 mM, about 60 mM, about 65 mM, about 70 mM, about 75 mM, about 80 mM, about 85 mM, about 90 mM, about 95 mM or about 100 mM, including any ranges or values within these ranges.

In one embodiment, the ionic excipient is present at a concentration of about 50 mM to about 125 mM.

In one embodiment, the ionic excipient is present at a concentration of about 50 mM to about 100 mM.

In one embodiment, the ionic excipient is present at a concentration of about 75 mM to about 100 mM.

In suitable embodiments, the salt is NaCl, for example at a concentration of about 50 mM to about 100 mM, suitably at a concentration of about 70 mM.

In suitable embodiments, the salt is arginine hydrochloride, for example at a concentration of about 50 mM to about 100 mM, suitably at a concentration of about 80 mM.

### Buffers

The formulations described herein suitably comprise one or more buffers. As used herein, "buffer" refers to an excipient for maintaining the pH of a formulation. Exemplary buffers for use in the formulations provided herein include, but are not limited to histidine, histidine hydrochloride (histidine HCl), sodium succinate, sodium acetate, sodium acetate/acetic acid, sodium phosphate, citrate, phosphate, succinate, glycine, and acetate. In one embodiment, the buffer for use in the formulations described herein is sodium acetate/acetic acid. In one embodiment, the one or more buffers is a buffer comprising histidine. In one embodiment, the one or more buffers are selected from a buffer comprising histidine succinate, histidine acetate, histidine citrate, histidine chloride or histidine sulfate. In one embodiment, the one or more buffers is histidine, histidine hydrochloride, or a combination thereof (histidine/ histidine hydrochloride). In one embodiment, the one or more buffers is L-histidine/ L-histidine hydrochloride monohydrate.

The concentration of a buffer, suitably sodium acetate/acetic acid, in the pharmaceutical formulations described herein is generally in the range of about 10 mM to about 100 mM, more suitably about 15 mM to about 80 mM, about 25 mM to about 75 mM, about 30 mM to about 60 mM, about 40 mM to about 60 mM, about 40 mM to about 50 mM, or about 15 mM, about 20 mM, about 25 mM, about 30 mM, about 35 mM, about 40 mM, about 45 mM, about 50 mM, about 55 mM, about 60 mM, about 65 mM, about 70 mM or about 75 mM, including any ranges or values within these ranges.

In one embodiment, the one or more buffers is L-histidine/ L-histidine hydrochloride monohydrate, for example at a concentration of about 10 mM to about 50 mM, suitably at a concentration of about 30 mM.

The pH of the buffer is preferably in the range of pH 5.5 to pH 6.0.

It will be understood that a buffer may, itself, be an ionic excipient. Thus, in one embodiment, the buffer is the ionic excipient. In this embodiment, the concentration of the buffer should be above 50 mM i.e. in line with the concentration of the ionic excipient disclosed herein. Preferable concentrations for the buffer in this embodiment are as discussed anywhere herein in relation to the ionic excipient.

Put another way, in one embodiment, the ionic excipient also acts as a buffer in the formulation. In this embodiment, an additional buffer may or may not be present.

### Sugars and Surfactants

The formulations described herein suitably comprise a sugar, for example, but not limited to, trehalose, lactose, mannitol, mellibiose, melezitose, raffinose, mannotriose, stachyose and sucrose. In other embodiments, a polyol such as trihydric or higher molecular weight sugar alcohols, e.g. glycerin, dextran, erythritol, glycerol, arabitol, xylitol, sorbitol, and mannitol, can be used. Examples of reducing sugars include, but are not limited to, glucose, maltose, maltulose, iso-maltulose and lactulose. Examples of non-reducing sugars include, but are not limited to, trehalose, non-reducing glycosides of polyhydroxy compounds selected from sugar alcohols and other straight chain polyalcohols. Examples of sugar alcohols include, but are not limited to, monoglycosides, compounds obtained by reduction of disaccharides such as lactose, maltose, lactulose and maltulose. The glycosidic side group can be either glucosidic or galactosidic. Additional examples of sugar alcohols include, but are not limited to, glucitol, maltitol, lactitol and iso-maltulose. In one embodiment, the sugar is selected from the group consisting of trehalose, lactose, mannitol, raffinose and sucrose. In specific embodiments, trehalose is used as a sugar in the formulations described herein. In specific embodiments, sucrose is used as a sugar in the formulations described herein.

Suitably, the amount of sugar, for example trehalose, in a formulation described herein is about 1 % (w/v) to about 10 % (w/v). Unless otherwise noted, percentage of a component (%) is used herein indicate a weight/volume (w/v) %. In exemplary embodiments, the amount of sugar in a pharmaceutical formulation described herein is about 1 % (w/v) to about 8 % (w/v), or about 2 % (w/v) to about 6 % (w/v), about 2 % (w/v) to about 5 % (w/v), about 3 % (w/v) to about 5 % (w/v), or about 1 % (w/v), about 2 % (w/v), about 3 % (w/v), about 4 % (w/v), about 5 % (w/v), about 6 % (w/v), about 7 % (w/v), about 8 % (w/v), about 9 % (w/v), or about 10 % (w/v), including any values and ranges within these ranges.

The formulations described herein suitably comprise a surfactant.

The term "surfactant" as used herein refers to organic substances having amphipathic structures; namely, they arc composed of groups of opposing solubility tendencies, typically an oil- soluble hydrocarbon chain and a water-soluble ionic group. Surfactants can be classified, depending on the charge of the surface-active moiety, into anionic, cationic, and nonionic surfactants. Surfactants are often used as wetting, emulsifying, solubilizing, and dispersing agents for various pharmaceutical formulations and preparations of biological materials. Pharmaceutically acceptable surfactants like polysorbates (e.g. polysorbates 20, 40, 60 or 80); polyoxamers (e.g. poloxamer 188); Triton; sodium octyl glycoside; lauryl-, myristyl-, linoleyl- , or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. lauroamidopropyl); myristamidopropyl-, palmidopropyl-, or isostearamidopropyl-dimethylamine; sodium methyl cocoyl-, or disodium methyl oleyl-taurate; and the MONAQUA ™ series (Mona Industries, Inc., Paterson, N. J.), polyethyl glycol, polypropyl glycol, and copolymers of ethylene and propylene glycol (e.g. Pluronics, PF68 etc), can be used in the pharmaceutical formulations described herein. Suitably the surfactant is a polysorbate, including for example, polysorbate-20, polysorbate-40, polysorbate-60, and polysorbate-80. In one embodiment, the surfactant is polysorbate-80.

Suitably, the formulations described herein comprise a surfactant (suitably polysorbate-80) at about 0.001 % to about 0.5 % (w/v), more suitably about 0.002 % to about 0.1 % of a surfactant, for example about 0.01 % to about 0.2 %, about 0.02 % to about 0.01 %, about 0.02 % to about 0.07 %, about 0.03 % to about 0.06 %, about 0.04 % to about 0.06 %, or about 0.02 %, about 0.025 %, about 0.03 %, about 0.035 %, about 0.04 %, about 0.045 %, about 0.05 %, about 0.055 %, about 0.060 %, about 0.065 %, about 0.07 %, about 0.075 %, about 0.08 %, about 0.085 %, about 0.09 %, about 0.095 %, or about 0. 1% of a surfactant, including any ranges or values within these ranges.

The formulations described herein suitably comprise a surfactant and a sugar. The formulations described herein suitably comprise a surfactant and one or more buffers. The formulations described herein suitably comprise a sugar and one or more buffers. The formulations described herein suitably comprise a surfactant, a sugar, and one or more buffers.

The formulations described herein can also include one or more additional excipients, including for example, one or more sugars, salts, amino acids, polyols, chelating agents, emulsifiers and/or preservatives.

### Pharmaceutical Use

The formulations of the invention preferably are pharmaceutical formulations. Suitably, the pharmaceutical formulations described herein are "pharmaceutically acceptable," and thus would meet the necessary approval requirements required by a regulatory agency of the Federal or a state government, or listed in the U.S. Pharmacopeia, European Pharmacopeia, or other generally recognized pharmacopeia, so as to be used in animals, and more particularly in humans.

The present invention provides a pharmaceutical formulation as described anywhere herein for use as a medicament. The present invention provides a pharmaceutical formulation as described anywhere herein for use in the treatment of a disease. The present invention provides a method of treating a disease in a subject comprising administering a pharmaceutical formulation as described anywhere herein to the subject. Also provided herein are methods of treating a subject by administering a therapeutically effective amount of a pharmaceutical formulation as described anywhere herein to the subject.

As used herein, the term "subject" includes any human or nonhuman animal. The term "nonhuman animal" includes all vertebrates, for example, but not limited to, mammals and non-mammals, such as nonhuman primates, sheep, dogs, cats, horses, cows, chickens, amphibians, reptiles, etc. In one embodiment, the subject is a human.

The disease may be selected from the group consisting of diabetes, cardiovascular disease, infectious disease, rheumatoid arthritis, vasculitis, giant cell arthritis, glomerular nephropathy, lupus nephritis, uveitis, atopic dermatitis, cirrhosis, psoriatic arthritis, chronic obstructive pulmonary disease, severe asthma, neutrophilic asthma, and myeloid leukemia.

In embodiments, the formulation is administered to a subject subcutaneously or by injection.

Suitably, the formulations arc a liquid formulation or a frozen formulation.

Also provided herein are methods of preparing a pharmaceutical formulation comprising preparing a pharmaceutical formulation as described herein, and suitably loading the pharmaceutical formulation into a syringe to form a pre-filled syringe.

Suitably, the pharmaceutical formulations described herein are prepared in sterile water, or are resuspended in sterile water for injection at the desired volume.

In exemplary embodiments, the pharmaceutical formulations have a volume of about 0.1 mL to about 20.0 mL, more suitably about 0.5 mL to about 15.0 mL, about 0.5 mL to about 12.0 mL, about 1.0 mL to about 10.0 mL, about 1.0 mL to about 5.0 mL, about 1.0 mL to about 2.0 mL or about 0.5 mL, about 0.6 mL, about 0.7 mL, about 0.8 mL, about 0.9 mL, about 1.0 mL, about 1.1 mL, about 1.2 mL, about 1.3 mL, about 1.4 mL, about 1.5 mL, about 1.6 mL, about 1.7 mL, about 1.8 mL, about 1.9 mL, about 2.0 mL, about 2.1 mL, about 2.2 mL, about 2.3 mL, about 2.4 mL, about 2.5 mL, about 2.6 mL, about 2.7 mL, about 2.8 mL, about 2.9 mL, or about 3.0 mL, including any ranges or values within these ranges.

While in suitable embodiments, the pharmaceutical formulations described herein are liquid formulations, i.e., pharmaceutical formulations prepared in sterile water or water for injection (WFI), the pharmaceutical formulations can also be frozen formulations or previously lyophilized formulations.

The present invention also provides a lyopliilized cake which is capable of being reconstituted using only sterile water into a formulation according to the invention as described herein. It will be understood that the ratio of antibody: ionic excipient will be the same in the lyophilized cake as in the post-lyophilized formulation. In one embodiment, the molar ratio of ionic excipient:antibody is in the range 450:1 to 40:1. Where the formulation has been lyophilized, the concentrations provided herein for the formulation are the post-reconstitution concentrations and thus are the concentrations in the so-called 'drug product'. By way of example, if a half-reconstitution strategy is used (where half the volume of water removed during lyophilization is returned during reconstitution), then after reconstitution, the concentration of the antibody will be twice what it was prior to lyophilization i.e. twice what is was in the so-called pre-lyophilization 'drug-substance' composition. It will therefore be understood that the present invention further provides a composition capable of being lyophilized to form a lyophilized cake, wherein the lyophilized cake is capable of being reconstituted using only sterile water into a formulation according to the invention as described herein. Suitable reconstitution strategies will be known to those skilled in the art.

In embodiments, it is desirable to prepare frozen formulations by providing a liquid pharmaceutical formulation as described herein, and freezing the formulation under appropriate conditions. For example, the frozen formulations can be provided by freezing the liquid formulations to less than 0 °C, more suitably to about -20 °C, about - 40 °C, about -60 °C, or suitably to about -80 °C. The pharmaceutical formulations are also suitably prepared as liquid formulations and stored at about 2 °C to about 8 °C, or about 2 °C, about 3 °C, about 4 °C, about 5 °C, about 6 °C, about 7 °C or about 8 °C.

Suitable protocols and methods for preparing lyophilized pharmaceutical formulations from liquid and/or frozen formulations are known in the art.

### Stability of Formulations

In exemplary embodiments, the formulations described herein are stable for extended periods of storage at room temperature or at a temperature range of about 2 °C to about 8 °C, suitably about 5 °C. As used herein, room temperature is generally in the range of about 22 °C to about 25 °C. Suitably the pharmaceutical formulations are stable after storage at about 2 °C to about 8 °C (e.g. 5 °C) for at least six (6) months. As used herein, the term "stable" for a period of storage (or "stability") is used to indicate that the monoclonal antibody, suitably IgG4 monoclonal antibody, pharmaceutical formulations resist aggregation, degradation, half antibody formation, and/or fragmentation. The stability of the monoclonal antibodies can be assessed by degrees of aggregation, degradation, half antibody formation or fragmentation, as measured by high performance size exclusion chromatography (HPSEC), static light scattering (SLS), Fourier Transform Infrared Spectroscopy (FTIR), circular dichroism (CD), urea unfolding techniques, intrinsic tryptophan fluorescence, differential scanning calorimetry, and/or ANS binding techniques, compared to a reference.

The overall stability of a pharmaceutical formulation comprising monoclonal antibodies can be assessed by various immunological assays including, for example, ELISA and radioimmunoassay using isolated antigen molecules.

The phrase "low to undetectable levels of aggregation" as used herein refers to pharmaceutical formulations containing no more than about 5 %, no more than about 4 %, no more than about 3 %, no more than about 2 %, no more than about 1 %, or no more than about 0.5 % aggregation by weight of protein as measured by high performance size exclusion chromatography (HPSEC) or static light scattering (SLS) techniques. Suitably, the pharmaceutical formulations exhibit ≤ 5.0 % aggregation, more suitably ≤ 4.0 % aggregation, ≤ 3.0 % aggregation, ≤ 2.0 % aggregation, ≤ 1.0 % aggregation, or 0.5 % aggregation. Suitably, the liquid pharmaceutical formulations and/or frozen pharmaceutical formulations exhibit ≤ 5.0 % aggregation, more suitably ≤ 4.0 % aggregation, ≤ 3.0 % aggregation, ≤ 2.0 % aggregation, ≤ 1.0 % aggregation, or 0.5 % aggregation.

The term "low to undetectable levels of fragmentation" as used herein refers to pharmaceutical formulations containing equal to or more than about 80 %, about 85 %, about 90 %, about 95 %, about 98 %, or about 99 % of the total monoclonal antibody, for example, in a single peak as determined by HPSEC, or reduced Capillary Gel Electrophoresis (rCGE), representing the non-degraded monoclonal antibody, or a non-degraded fragment thereof, and containing no other single peaks having more than about 5 %, more than about 4 %, more than about 3 %, more than about 2 %, more than about 1 %, or more than about 0.5 % of the total monoclonal antibody. Fragmentation may be measured suitably in IgG4 monoclonal antibodies. Without wishing to be bound by theory, it is thought that decreased self-aggregation is due to improved colloidal stability, as evidenced by increased kD value. In exemplary embodiments, the formulations described herein have reduced opalescence and decreased phase separation as detected by visual observation, light scattering, nephelometry or turbidimetric methods.

Further embodiments, features, and advantages of the embodiments, as well as the structure and operation of the various embodiments, are described in detail below with reference to accompanying drawings.

### EXAMPLES

### EXAMPLE 1 - IgG4 Formulations

The formulation challenge of antibodies with low or neutral pI values is that when these antibodies are formulated at a pH outside the range of 5.5 to 7.5 (as generally needed to increase protein charge and colloidal stability), additional instabilities are observed. At acidic pH an increased rate of fragmentation, reduced conformational stability and increased aggregation are observed. At basic pH the potential for increased oxidation, deamidation and fragmentation and incompatibility with glass containers are present. IgG4 antibodies are particularly useful for studying such instabilities since IgG4 antibodies typically have low or neutral pI values.

The following details the methods used to develop the formulations described herein that have optimized the storage stability of monoclonal antibodies that possess a neutral pI, and also overcomes the neutral pH physical instabilities mentioned above.

As described herein, the formulations have a combination of neutral pH and an ionic excipient such as a salt. Optionally a sugar may also be used in the formulations and this has been shown to provide further improvements in some cases.

The sugar (sucrose and trehalose are examples) can (in combination with an ionic excipient) further increase conformational stability; and the ionic excipient (sodium chloride, lysine hydrochloride and arginine hydrochloride for example) increases the colloidal stability of the monoclonal antibody molecules. Furthermore, the neutral pH (about pH 6) of the formulations also minimizes the acidic and basic degradation pathways described above. These formulations provide superior overall storage stability for these monoclonal antibodies. Other suitable components of the formulations are a buffer with a neutral pKa (e.g., NaAC, histidine HCl, sodium phosphate).

As described herein, and listed in Table 1, CAM-3001 (an anti-GM-CSFRa monoclonal antibody), two anti-IL-13 monoclonal antibodies, and an anti-C5/C5a monoclonal antibody were chosen for this study as they are IgG4's with neutral pI values.

**TABLE 1: IgG4 Molecules Studied**

| **Molecule** | **Type** | **pI** |
|---|---|---|
| CAM-3001 (anti-GM-CSF-Rα) | IgG4 | 6.7 |
| anti-IL13 mAb A (CAT-354) | IgG4 | 7.4 |
| anti-C5/C5a mAb (MEDI7814) | IgG4-P | 6.8-7.3 |
| anti-IL-13 mAb B | IgG4-P | 6.6 |

CAT-354 is a human antibody of the IgG4 subclass that specifically binds the human interleukin 13 (IL-13), blocking interactions with the IL-13 receptor. The DNA and derived amino-acid sequences of the light chain and heavy chain for CAT-354 are provided in **Error! Reference source not found.** and **Error! Reference source not found.,** respectively. In **Error! Reference source not found.** and **Error! Reference source not found.,** the V_{H} and V_{L} sequences are labelled and the 6 CDRs are underlined. The CAT-354 molecule is a human monoclonal IgG4 (lambda light-chain) antibody with a molecular weight of approximately 147,000 Daltons (Da) (including oligosaccharides). The antibody is composed of two identical heavy chains of approximately 49,500 Da each, and two identical light chains of approximately 22,500 Da each. CAT-354 contains fucosylated biantennary complex and high mannose N-linked carbohydrates attached to each heavy chain at Asn-299. The average size of the oligosaccharide moiety is approximately 1,650 Da per heavy chain.

CAM-3001 is an antibody which binds to Granulocyte Macrophage Colony Stimulating Factor Receptor alpha (GM-CSFRα). CAM-3001 is disclosed in International Patent Application Publication WO 2007/110631, the disclosure of which is incorporated by reference herein. The heavy chain CDRs (HCDRs) of CAM-3001 are disclosed as SEQ ID NOs: 53-55 in WO 2007/110631. The light chain CDRs (LCDRs) of CAM-3001 are disclosed as SEQ ID NOs: 58-60 in WO 2007/110631. The heavy chain variable region (VH) of CAM-3001 is disclosed as SEQ ID NO: 52 in WO 2007/110631. The light chain variable region (VL) of CAM-3001 is disclosed as SEQ ID NO: 218 in WO 2007/110631.

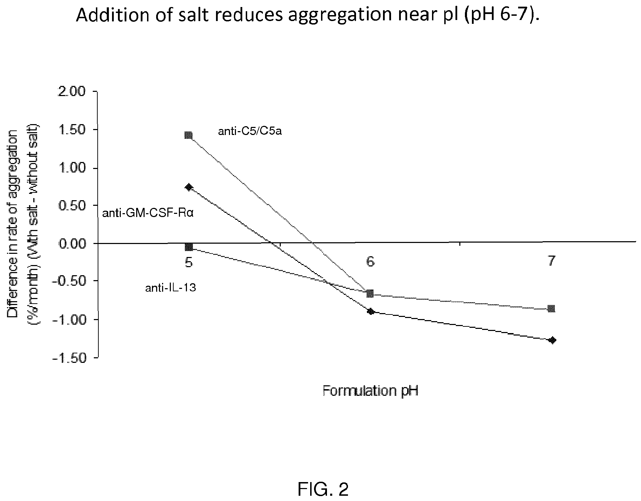

As shown in FIG. 2, in the presence of salt at a concentration of 100 mM, the aggregation rate increases when the molecule is at pH 5, but the aggregation rate is reduced when the molecule is at pH 6 and pH 7.

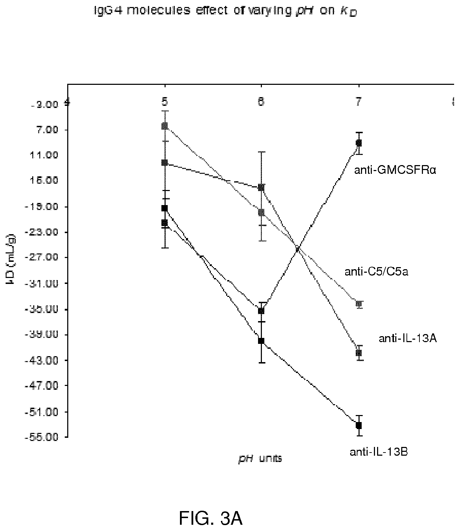

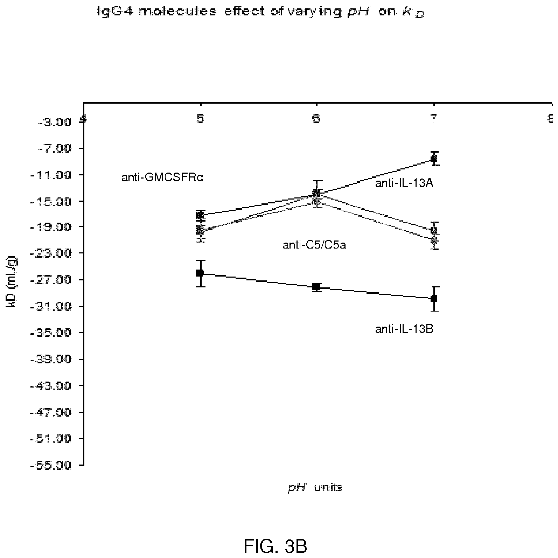

The correlation between solution properties of monoclonal antibodies with the diffusion interaction parameter (k_{D}) was examined by testing a combination of conformation stability and colloidal stability in order to achieve the most stable formulation. The k_{D} was measured as an indication of colloidal stability, while differential scanning calorimetry (DSC) was examined as a measure of conformational stability. FIG. 3 shows the k_{D} at varying pH for the monoclonal antibodies studied. FIG. 3B shows the k_{D} at varying pH for the monoclonal antibodies studied after adding salt at a concentration of 100 mM. At low ionic strength, the k_{D} generally becomes more negative with increasing pH. The addition of salt makes the k_{D} less negative for all molecules studied.

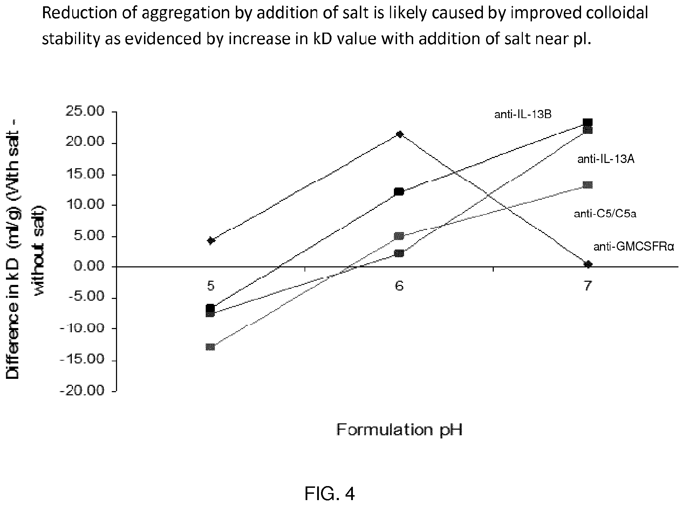

In the results depicted in FIG. 4, salt was added to the formulations at a concentration of 100 mM and the colloidal stability examined. Note generally a reduction in colloidal stability at pH 5, but an increase in colloidal stability at pH 6 and pH 7.

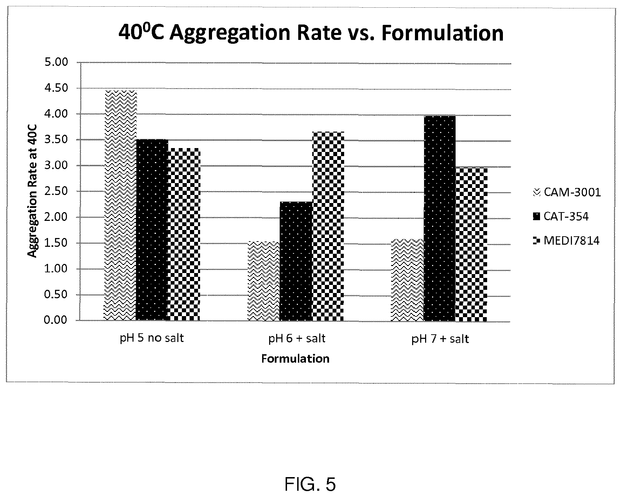

In the results depicted in FIG. 5, aggregation rate data at 40°C was obtained for formulations with or without 100 mM salt. Formulations with salt near pI (pH 6 or 7) show reduced aggregation compared to formulation without salt away from pI.

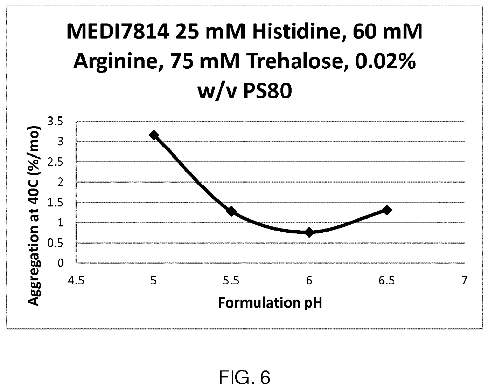

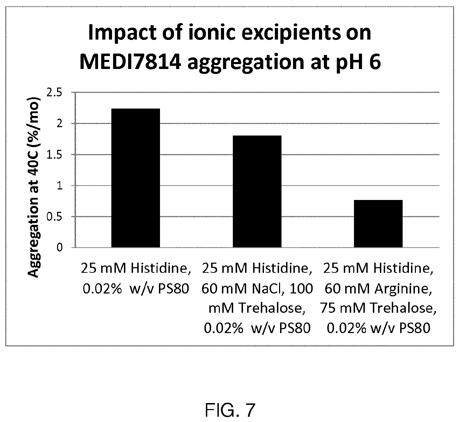

From the aggregation rate results depicted in FIG. 6 and 7, it can be seen that charged amino acids can also act as ionic stabilizers near the molecule pI and may in some cases be more stabilizing than NaCl.

The addition of salt to a histidine-based buffer was examined to determine the effect on the stability of IgG4. The following buffer systems were examined: Control buffer (25 mM histidine, 7 % sucrose, pH 6); Test buffer (25 mM histidine, 7 % sucrose; 100 mM NaCl, pH 6) and the impact of salt on the appearance are summarized in Table 2, below.

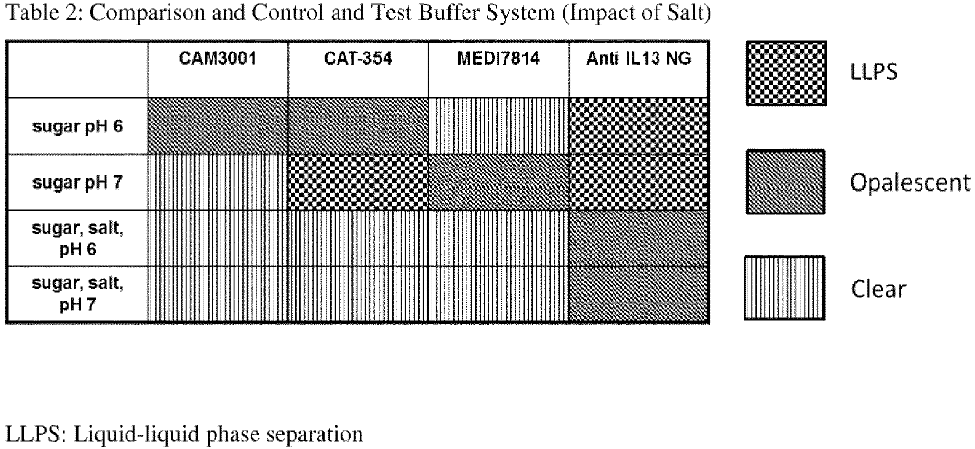

As demonstrated above, the addition of salt reduced the rate of aggregation, and the rate of monomer loss, and improved the appearance of the formulations.

In summary, formulations comprising salt and with or without sugar have lower opalescence, mitigated phase separation in some instances, and provides as good or better stability when compared to formulations comprising sucrose alone.

Freeze-thaw studies showed that no significant changes in product quality, such as subvisible particles, were seen after three freeze-thaw cycles. In summary, the addition of both, salt and sugar, improved the stability of the IgG4 molecules, even when formulated at pHs near their pIs.

### EXAMPLE 2 - IgG1 Formulation

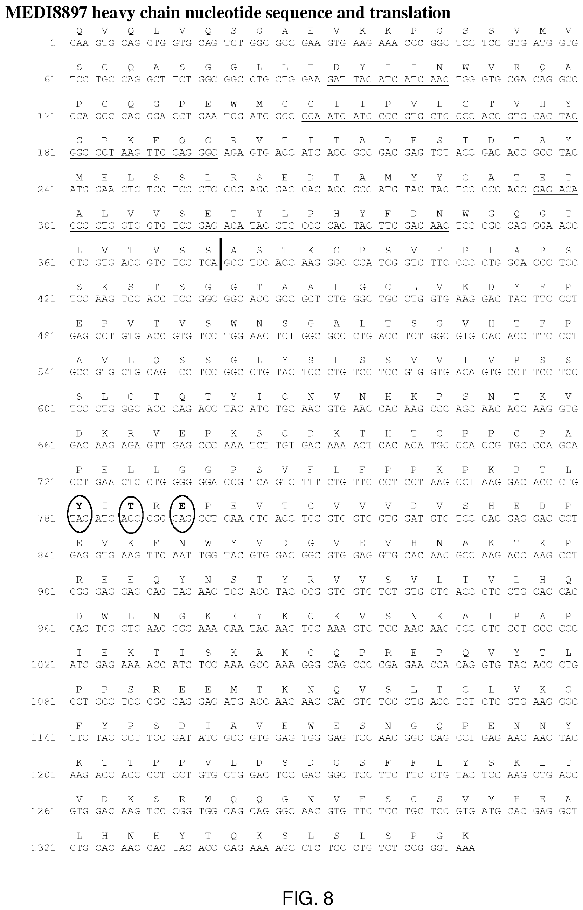

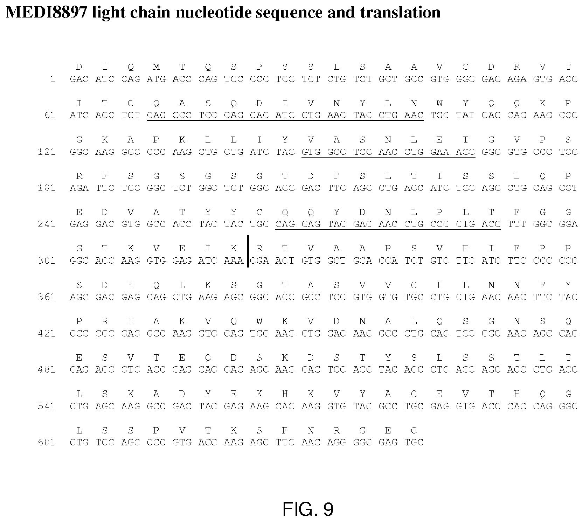

MEDI8897 is a human IgG1κ- YTE monoclonal antibody directed against RSV-F protein. Three amino acid substitutions (M252Y/S254T/T256E; called YTE) in the CH2 region of the Fc domain were introduced to increase the serum half-life of MED18897. Sequence information for MED18897 is provided in Figures 8 and 9. MED18897 pI was measured by cIEF to be 6.4-6.7 with the main peak at 6.4. The pI overlaps with the formulation buffer range (5.5-6.5) suggesting potential issues with manufacturing, formulation and storage stability.

MEDI8897 thermal stability was measured by differential scanning calorimetry. Tm1 was found to be 61°C while Tm2 was 82°C. A Tm1 greater than 50°C suggests that the molecule has acceptable colloidal stability.

### Stability Summary

Upon receipt of MEDI8897 in a standard buffer (25 mM Histidine, 7% sucrose, pH 6.0), phase separation was observed at 2 to 8°C. The supernatant layer had a protein concentration of 75 mg/ml while the bottom layer was 125 mg/ml. Upon equilibration at 25°C the two distinct phases disappeared and only one single phase was observed. The phase separation at 2 to 8°C was thought to be due to the pI of MEDI8897 which is close to the formulation pH of 6.0. A scouting study was initiated to find a more appropriate formulation buffer for MEDI8897 stability assessment, targeting a condition which maintained solubility and prevented phase separation of MEDI8897 at 100 mg/ml.

Formulating in the standard buffer (25 mM histidine, 7% sucrose) at pH's below 5.9 or above 6.7 mitigated phase separation. Addition of 75 mM NaCl to the standard buffer between pH 5.0 and 6.7 also mitigated phase separation. Finally, acetate and phosphate buffers at pH values away from the pI also mitigated phase separation. Based on these screening studies and previous knowledge of mAbs with pIs within the formulation space, an alternate buffer (25mM His/HisHCl, 75mM NaCl, 4% Sucrose, 0.02% PS80, pH 6.0) was selected for evaluation.

### kD Studies

For the first kD screen, all samples were evaluated in 25 mM Histidine pH 5.5 base buffer from 2-10 mg/ml at 25°C. This buffer was chosen in lieu of pH 6.0 because MEDI8897 is more soluble at pH 5.5, facilitating DLS measurements which are sensitive to insoluble particles. Ionic excipients including arginine-HCl, lysine-HCl and NaCl were evaluated at 10, 25, 50, 75 and 100 mM concentrations. In addition, proline, alanine, Na₂SO₄ and histidine were evaluated at the 100 mM concentration only. Finally, 2, 4, and 6% sucrose were evaluated to determine if sucrose influences protein-protein interactions. All conditions were compared to a buffer control (25 mM Histidine pH 5.5).

The control samples showed distinct protein-protein interactions, with the hydrodynamic radius increasing from 6.2 to 7.8 nm from 2-10 mg/ml. Arginine-HCl, lysine-HCl and NaCl showed reduction of protein-protein interactions (PPI) stalling at 25 mM concentrations as evidenced by no increase in hydrodynamic size over the 2-10 mg/ml concentration range. No additional effects were seen between 25 and 100 mM. At 100 mM concentration, proline and alanine showed PPI similar to the control while Na₂SO4 and Histidine mitigated PPI. Finally, sucrose concentration showed no impact on PPI. This data illustrates that charged excipients (Arg-HCl, Lys-HCl, Histidine and Na₂SO₄) mitigate protein-protein interactions while neutral excipients (sucrose, proline, alanine) do not mitigate PPI. Therefore, addition of ionic excipients at pH 5.5 reduced phase separation at 100 mg/ml.

### 40°C Stability Evaluation

Based on kD screening, several conditions were selected for 40°C stability evaluation. Table 3 summarizes the formulation conditions and 1 month degradation rates seen at 40°C.

**Table 3- 40°C Stability Rates, Formulation Screen 1- Excipient Screening**

| **Number** | **Excipient** | **Conc (mM)** | **% Mon/ mo** | **% Agg/mo** | **% Frag/mo** |
|---|---|---|---|---|---|
| 1 | NaCl | 25 | -5.9 | 4.2 | 1.8 |
| 2 | NaCl | 75 | -6.1 | 4.1 | 1.9 |
| 3 | NaCl | 95 | -5.4 | 3.5 | 1.9 |
| 4 | NaCl | 120 | -5.4 | 3.5 | 1.9 |
| 5 | Arg-HCl | 25 | -5.4 | 3.5 | 1.8 |
| 6 | Arg-HCl | 75 | -4.8 | 2.8 | 2.0 |
| 7 | Arg-HCl | 95 | -4.5 | 2.6 | 1.9 |
| 8 | Arg-HCl | 120 | -4.8 | 2.8 | 2.0 |
| 9 | Lys-HCl | 25 | -5.7 | 3.9 | 1.9 |
| 10 | Lys-HCl | 75 | -5.0 | 2.7 | 2.3 |
| 11 | Lys-HCl | 95 | -5.1 | 3.1 | 2.0 |

**Table 3- 40°C Stability Rates, Formulation Screen 1- Excipient Screening**

| **Number** | **Excipient** | **Conc (mM)** | **% Mon/ mo** | **% Agg/mo** | **% Frag/mo** |
|---|---|---|---|---|---|
| 12 | Lys-HCl | 120 | -4.9 | 2.9 | 2.0 |

| | | | | | |
|---|---|---|---|---|---|
| Base buffer for this study was 25 mM Histidine pH 6.0 | | | | | |

This study illustrates that arginine and lysine are more stabilizing than NaCl. In addition, 75 mM and above appears to stabilize against aggregation. Based on this study, arginine was selected as the most stabilizing lyo-friendly excipient and was moved forward to the next set of studies.

### Drug Product Stability on Final Lyo Cycle/ Representative Material

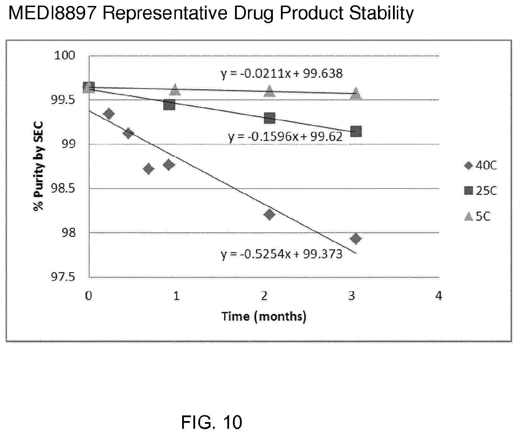

Stability of the drug product was evaluated. Three months of data was collected for the post reconstitution formulation of 100 mg/ml in 30 mM L-histidine/L-histidine hydrochloride monohydrate, 80 mM L-arginine hydrochloride, 120 mM sucrose, 0.04% (w/v) polysorbate 80, pH 6.0. Results are shown in Figure 10. Storage at 2-8°C showed virtually no change during the 3 month period, confirming the suitability of the formulation and lyo cycle for clinical use. These data thus demonstrate that the formulation provides appropriate stability and solubility and is suitable for first time in human clinical use.

**Table 4- Drug Product Stability 3 Month Data Summary**

| **Temperature** | **HIAC (≥10 µm)** | **HIAC (≥25 µm)** | **Bioassay** | **Recon Time** | **VI** | **KF** |
|---|---|---|---|---|---|---|
| 2-8°C | 216 | 108 | 97% | 2 min | < STD1 | 1.3% |
| 25 °C | 522 | 90 | 97% | 3 min | < STD1 | 1.4% |
| 40°C | 126 | 0 | 90% | 3 min | < STD2 | 1.7% |

### EXAMPLE 3- IgG4 formulation

Formulations of antibody MEDI578 were tested at pH6. MEDI578 is an IgG4 monoclonal antibody having a pI of 6.3 to 6.8.

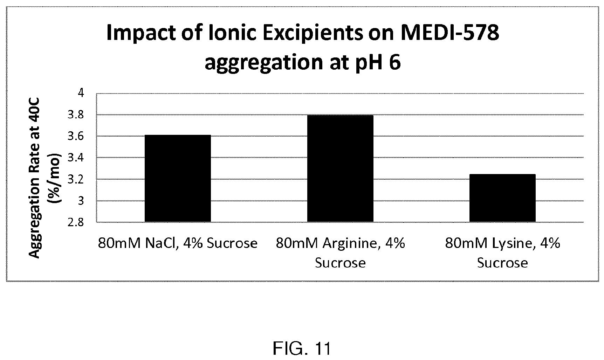

Results are shown in Figure 11.

### EXAMPLE 4- IgG4 formulation

All formulations that were tested contained 100mg/ml MEDI578, 20mM histidine pH 6.5 without polysorbate. MEDI578 is an IgG4 monoclonal antibody having a pI of 6.3 to 6.8.

**Table 5 - Formulation information**

| Form ulation # | Excipients | Tg' | Osmo (mOsm/kg) | Opalescence | Particles |
|---|---|---|---|---|---|
| 1 | 9% Sucrose | -25.8 | 387 | 4 | 2 |
| 2 | 9% Trehalose | -23.6 | 341 | 4 | 2 |
| 3 | 275mM Mannitol | -28.2 | 331 | 5 | 2 |
| 4 | 125mM Lys/35mM NaCl | -30.1 | 361 | 3 | 1 |
| 5 | 20mM Lys/8% sucrose | -25.7 | 392 | 3 | 1 |
| 6 | 50mM Lys/6% sucrose | -26.4 | 379 | 3 | 1 |
| 7 | 80mM Lys/4% sucrose | -27.3 | 326 | 3 | 1 |
| 8 | 80mM NaCl/4% sucrose | -26 | 365 | 3 | 1 |
| 9 | 80mM Arg/4% sucrose | -24.4 | 363 | 3 | 1 |
| 10 | 150mM Mannitol/4% sucrose | -27.8 | 379 | 5 | 2 |

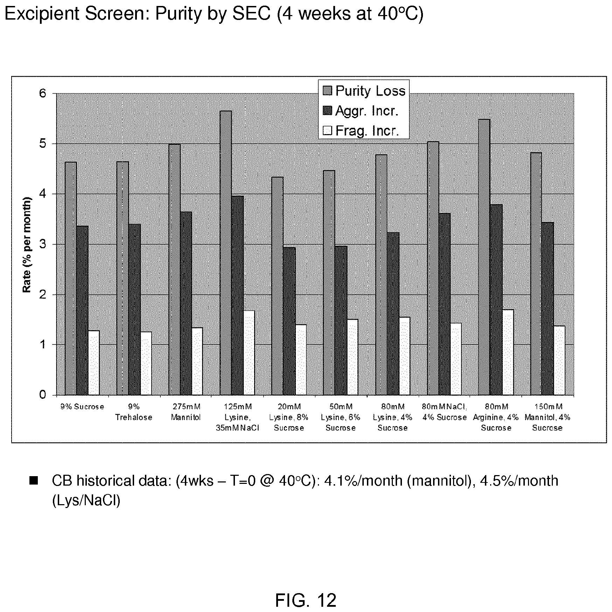

Figure 12 shows purity by size-exclusion chromatography.

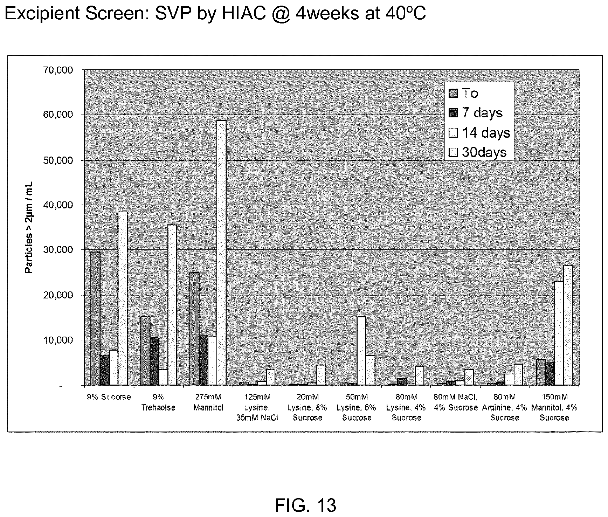

Figure 13 shows a reduction in subvisible particles in formulations containing ionic excipients under thermal stress conditions. Colloidal stability is improved.

All documents, patents, journal articles and other materials cited in the present application arc hereby incorporated by reference.

Although the present invention has been fully described in conjunction with several embodiments thereof with reference to the accompanying drawings, it is to be understood that various changes and modifications can be apparent to those skilled in the art. Such changes and modifications are to be understood as included within the scope of the present invention as defined by the appended claims, unless they depart there from.

The invention is further embodied by the following items:
1. A formulation comprising:
   i. a monoclonal antibody; and
   ii. an ionic excipient;
      wherein the monoclonal antibody is present at a concentration of about 50mg/ml or greater and the ionic excipient is present at a concentration of about 50 to about 150 mM and the formulation has a pH of 5.5 to 7.5.
2. A formulation comprising:
   i. a monoclonal antibody; and
   ii. an ionic excipient;
      wherein the monoclonal antibody is present at a concentration of about 50mg/ml or greater and the ionic excipient is present at a concentration of about 50 to about 150 mM and the formulation has a pH of 5.5 to7.5; and wherein the aggregation rate of the monoclonal antibody in the formulation is reduced compared to the aggregation rate of the same antibody in the same formulation but without an ionic excipient.
3. A formulation according to item 1 or 2, wherein the monoclonal antibody has a pi in the range of pH 5.5 to pH 7.5.
4. A formulation according to any one of items 1 to 3, wherein the monoclonal antibody has a pi in the range of pH 6.0 to pH 7.5.
5. A formulation according to any one of items 1 to 3, wherein the monoclonal antibody has a pi in the range of pH 6.4 to pH 7.5.
6. A formulation according to any one of items 1 to 5, wherein the monoclonal antibody is an IgG4 monoclonal antibody.
7. A formulation according to any one of items 1 to 6, wherein the monoclonal antibody is present in the formulation at a concentration of about 100 mg/ml to about 200 mg/ml.
8. A formulation according to item 7, wherein the monoclonal antibody is present in the formulation at a concentration of about 100 mg/ml.
9. A formulation according to any one of items 1 to 8, wherein the formulation has a pH in the range of about pH 5.5 to about pH 6.5.
10. A formulation according to any one of items 1 to 8, wherein the formulation has a pH in the range of about pH 5.7 to about pH 6.3.
11. A formulation according to any one of items 1 to 8, wherein the formulation has a pH in the range of about pH 5.7 to about pH 6.1.
12. A formulation according to item 11, wherein the formulation has a pH of about pH 6.0.
13. A formulation according to any one of items 1 to 12, wherein the ionic excipient is a salt.
14. A formulation according to item 13, wherein the salt is NaCl.
15. A formulation according to item 13, wherein the salt isarginine hydrochloride.
16. A formulation according to item 13, wherein the salt islysine hydrochloride.
17. A formulation according to any one of items 1 to 16, wherein the ionic excipient is present at a concentration of about 75 mM to about 100 mM.
18. A formulation according to item 17, wherein the ionic excipient is present at a concentration of about 80 mM.
19. A formulation according to any one of items 1 to 18, wherein the formulation further comprises a sugar.
20. A formulation according to item 19, wherein the sugar is trehalose.
21. A formulation according to item 19, wherein the sugar is sucrose.
22. A formulation according to any one of items 19 to 21, wherein the sugar is present at a concentration of about 100 mM to about 140 mM.
23. A formulation according to item 22, wherein the sugar is present at a concentration of about 120 mM.
24. A formulation according to any one of items 1 to 23, wherein the formulation further comprises one or more buffers.
25. A formulation according to item 24, wherein the one or more buffers is selected from histidine, histidine hydrochloride, and histidine/ histidine hydrochloride.
26. A formulation according to item 25, wherein the one or more buffers is L-histidine/ L-histidine hydrochloride monohydrate.
27. A formulation according to any one of items 24 to 26, wherein the one or more buffers is present at a concentration of about 10 mM to about 50 mM.
28. A formulation according to item 27, wherein the one or more buffers is present at a concentration of about 30 mM.
29. A formulation according to any one of items 1 to 28, wherein the formulation further comprises a surfactant.
30. A formulation according to item 29, wherein the surfactant is a polysorbate.
31. A formulation according to item 30, wherein the surfactant is polysorbate-80.
32. A formulation according to any one of items 29 to 31, wherein the surfactant is present in the formulation at a concentration from about 0.02 % (w/v) to about 0.07 % (w/v).
33. A formulation according to item 32, wherein the surfactant is present in the formulation at a concentration of about 0.04% (w/v).
34. A formulation according to any one of items 1 to 33, wherein the formulation further comprises one or more additional excipients, including for example, one or more sugars, salts, amino acids, polyols, chelating agents, emulsifiers and/or preservatives.
35. A formulation according to item 1 comprising 150 mg/ml anti-IL-13 IgG4 antibody, 50 mM Sodium Acetate, 85 mM sodium chloride and 0.01% Polysorbate 80, wherein the formulation has a pH of pH 5.5.
36. A formulation according to item 35, wherein the antibody has the 6 CDR sequences of CAT-354 as shown in Figure 1-1 and Figure 1-2.
37. A formulation according to item 35, wherein the antibody has the VH and VL sequences of CAT-354 as shown in Figure 1-1 and Figure 1-2.
38. A formulation according to item 1 comprising 150 mg/ml anti-GM-CSF-Ra IgG4 antibody, 50mM sodium acetate/acetic acid, 106mM trehalose dehydrate, 70mM sodium chloride, and 0.05% (w/v) Polysorbate 80, wherein the formulation has a pH of pH 5.8.
39. A formulation according to item 38, wherein the antibody has the 6 CDR sequences of CAM-3001.
40. A formulation according to item 38, wherein the antibody has the VH and VL sequences of CAM-3 001.
41. A formulation according to any one of items 1 to 40, which is a pharmaceutical formulation.
42. A pharmaceutical formulation according to item 41 for use as a medicament.
43. A pharmaceutical formulation according to item 41 for use in the treatment of a disease.
44. A method of treating a disease in a subject comprising administering a pharmaceutical formulation according to item 41 to the subject.
45. Alyophilized cake capable of being reconstituted using only sterile water into a formulation as defined in any one of items 1 to 40 or a pharmaceutical formulation as defined in any one of items 41-43.
46. A formulation capable of being lyophilized to form a lyophilized cake, wherein the lyophilized cake is capable of being reconstituted using only sterile water into a formulation as defined in any one of items 1 to 40 or a pharmaceutical formulation as defined in any one of items 41-43.

## Claims

1. A formulation comprising:
i. an anti-GM-CSFRα antibody; and
ii. an ionic excipient;
wherein the anti-GM-CSFRα antibody has a pI in the range of pH 6.0 to pH 7.5 and the VH and VL sequences of CAM-3001, and
wherein the anti-GM-CSFRα antibody is present at a concentration of about 50 mg/ml or greater and the ionic excipient is present at a concentration of about 50 to about 150 mM and the formulation has a pH of 5.5 to 7.5.

2. The formulation of claim 1, wherein the aggregation rate of the monoclonal antibody in the formulation is reduced compared to the aggregation rate of the same antibody in the same formulation but without an ionic excipient.

3. The formulation according to claim 1 or 2, wherein the anti-GM-CSFRa antibody has a pI in the range of pH 6.4 to pH 7.5.

4. The formulation according to any one of claims 1 to 3, wherein the anti-GM-CSFRα antibody is an IgG4 monoclonal antibody.

5. The formulation according to any one of claims 1 to 4, wherein the anti-GM-CSFRα antibody is present in the formulation at a concentration of about 100 mg/ml to about 200 mg/ml.

6. The formulation according to claim 5, wherein the monoclonal antibody is present in the formulation at a concentration of about 100 mg/ml or of about 150 mg/ml.

7. The formulation according to any one of claims 1 to 6, wherein the formulation has a pH in the range of about pH 5.5 to about pH 6.5.

8. The formulation according to claim 7, wherein the formulation has a pH of about pH 5.8.

9. The formulation according to any one of claims 1 to 8, wherein the ionic excipient is a salt, optionally, wherein the salt is NaCl, arginine hydrochloride, or lysine hydrochloride.

10. The formulation according to any one of claims 1 to 9, wherein the ionic excipient is present at a concentration of about 50 mM to about 100 mM, optionally wherein the ionic excipient is present at concentration of 70 mM.

11. The formulation according to any one of claims 1 to 10, wherein the formulation further comprises a sugar, optionally, wherein the sugar is trehalose, sucrose, and wherein the sugar is present at concentration of about 100 mM to about 140 mM.

12. The formulation according to any one of claims 1 to 11, wherein the formulation further comprises one or more buffers, optionally, wherein the one or more buffers is selected from histidine, histidine hydrochloride, and histidine/ histidine hydrochloride.

13. The formulation according to any one of claims 1 to 12, wherein the formulation further comprises a surfactant, optionally, wherein the surfactant is polysorbate or polysorbate-80, and wherein the surfactant is present in the formulation at a concentration from about 0.02 % (w/v) to about 0.07 % (w/v).

14. The formulation according to claim 1, comprising 150 mg/ml anti-GM-CSFRa antibody, 50mM sodium acetate/acetic acid, 106 mM trehalose dehydrate, 70mM sodium chloride, and 0.05% (w/v) Polysorbate 80, wherein the formulation has a pH of pH 5.8.

15. The pharmaceutical formulation according to any one of claims 1-14 for use as a medicament or for use in the treatment of a disease.
